(19) Europäisches Patentamt / European Patent Office / Office européen des brevets

(11) **EP 4 763 230 A1**

(12) **EUROPEAN PATENT APPLICATION**
published in accordance with Art. 153(4) EPC

(43) Date of publication:
**24.06.2026 Bulletin 2026/26**

(21) Application number: **24858616.6**

(22) Date of filing: **28.08.2024**

(51) International Patent Classification (IPC):
$A61K\ 48/00^{(2006.01)}$    $A61K\ 47/26^{(2006.01)}$
$A61K\ 47/02^{(2006.01)}$    $A61K\ 38/37^{(2006.01)}$
$A61K\ 9/08^{(2006.01)}$    $A61P\ 7/04^{(2006.01)}$
$A61K\ 47/10^{(2017.01)}$

(86) International application number:
**PCT/CN2024/115096**

(87) International publication number:
**WO 2025/045082 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.09.2023 CN 202311123131**

(71) Applicant: **Kanglin Biotech (Hangzhou) Co., Ltd.**
**Hangzhou, Zhejiang 310018 (CN)**

(72) Inventors:
- **WU, Haoquan**
  **Hangzhou, Zhejiang 310018 (CN)**
- **JIANG, Chao**
  **Hangzhou, Zhejiang 310018 (CN)**
- **LI, Xinran**
  **Hangzhou, Zhejiang 310018 (CN)**
- **DAI, Yong**
  **Hangzhou, Zhejiang 310018 (CN)**
- **HU, Xiaodong**
  **Hangzhou, Zhejiang 310018 (CN)**
- **SU, Lingling**
  **Hangzhou, Zhejiang 310018 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

Remarks:
The complete document including Reference Table(s) and the Sequence Listing(s) can be downloaded from the EPO website

(54) **BUFFER SYSTEM, LIQUID PREPARATION CORRESPONDING THERETO, AND USE THEREOF**

(57) Provided are a buffer system for improving the stability and dispersibility of virus particles, a liquid preparation corresponding thereto, and a use thereof. The buffer system comprises a soluble salt substance, a polyol, and a pH buffer. The liquid preparation comprises a therapeutically effective amount of virus particles and the buffer system. The buffer system can significantly improve the stability and dispersibility of virus particles and reduce the content of aggregates and free nucleic acids. The signal response intensity of virus particles is not significantly reduced, viral activity is not significantly reduced, and the number of dispersed virus particles and the stability of the virus particles can be significantly improved (e.g., enhanced).

Effect of Different Formulations on the Thermal Stability of AAV

Figure 1

**Description**

**PRIORITY CLAIM**

The present disclosure claims priority to Chinese Patent Application No. 2023111231314, filed on September 1, 2023. The entire content of the aforementioned Chinese Patent Application is incorporated herein by reference.

**TECHNICAL FIELD**

The present disclosure relates to the field of biotechnology, and in particular, to a buffer system, a liquid formulation based thereon, and use thereof.

**BACKGROUND ART**

AAV has emerged as a novel tool for human gene therapy. Currently, during manufacturing, storage, transportation, and administration, AAV is subjected to repeated freeze-thaw cycles, refrigerated storage, or even room-temperature storage. These conditions pose a significant challenge to the stability of AAV and result in reduced transduction efficiency. Meanwhile, as the application scenarios of AAV expand beyond local low-dose administration, more attention should be paid to the adverse reactions associated with high-dose systemic administration. In recent years, the viral titers of marketed products have generally remained at approximately $1 \times 10^{13}$ vg/mL or even lower, and the osmolality of the solutions has been maintained at above 350 mOsm/kg or even higher, such as Glybera developed by Uniqure and Roctacian developed by BioMarin. Such characteristics are not conducive to high-dose systemic administration, may induce unnecessary immune responses, substantially increase the frequency of administration, and cause distress to patients.

Clinical research and application of AAV as a gene therapy vector have been increasing. For example, to avoid hepatotoxicity and the potential risk of liver cancer, hemophilia gene therapy must explore safer and more effective AAV vectors and routes of administration, change the tissue distribution of AAV-based drugs in the human body, and thus develop them into gene therapy drugs with significant safety advantages.

Against the backdrop of the increasing clinical research and application of AAV as a gene therapy vector, the current challenge lies in that the choice of excipients and buffers is severely limited by development and manufacturing, long-term stability, and administration routes. Therefore, there is an urgent need to develop and design an AAV formulation that balances stability, is suitable for various administration routes and high-concentration dosing, and ensures low osmolality.

**CONTENT OF THE INVENTION**

The objective of the present disclosure is to provide an AAV formulation that balances stability, is suitable for various administration routes and high-concentration dosing, and ensures low osmolality.

In a first aspect, the present disclosure provides a buffer system, wherein the buffer system comprises a soluble salt substance, a polyol, and a pH buffer.

In another alternative embodiment, the soluble salt substance comprises a soluble monovalent metal salt.

In another alternative embodiment, the soluble monovalent metal salt comprises a soluble sodium salt.

In another alternative embodiment, the soluble salt substance comprises a soluble divalent metal salt.

In another alternative embodiment, the soluble divalent metal salt comprises one or more of a soluble calcium salt, a soluble magnesium salt, a soluble zinc salt, a soluble copper salt, a soluble manganese salt, and a soluble chromium salt.

In another alternative embodiment, the polyol is selected from sucrose, mannitol, and glycerol.

In another alternative embodiment, the pH of the buffer system is maintained at 7.0-8.0 (e.g., 7.0-7.6 or 7.0-7.4).

In another alternative embodiment, the pH buffer is any one or more selected from the following buffers: PB buffer and HEPES buffer.

In another alternative embodiment, the polyol is sucrose.

In another alternative embodiment, the polyol is mannitol.

In another alternative embodiment, the polyol is glycerol.

In another alternative embodiment, the divalent metal ion in the soluble divalent metal salt is selected from $Ca^{2+}$, $Mg^{2+}$, $Zn^{2+}$, $Cu^{2+}$, $Mn^{2+}$, and $Cr^{2+}$.

In another alternative embodiment, the monovalent metal ion in the soluble monovalent metal salt comprises $Na^+$.

In another alternative embodiment, the $Na^+$ is derived from NaCl.

In another alternative embodiment, the $Ca^{2+}$ is derived from $CaCl_2$, $CH_3COOCa$.

In another alternative embodiment, the $Mg^{2+}$ is derived from $MgCl_2$, $Mg_2SO_4$.

In another alternative embodiment, the buffer system further comprises a surfactant.

In another alternative embodiment, the surfactant is selected from the following: F68, Tween20, or a combination thereof.

In another alternative embodiment, the concentration of the soluble monovalent metal salt in the soluble salt substance is 50-250 mM, e.g., 80-200 mM, 100-190 mM, or 100-160 mM.

In another alternative embodiment, the concentration (w/v) of the polyol is 0.5%-20%, e.g., 0.8%-10% or 0.9%-5%, based on the total weight/volume percentage of the buffer system.

In another alternative embodiment, the concentration (w/v) of the surfactant is 0.0005%-0.002%, e.g., 0.0008%-0.002%, based on the total weight/volume percentage of the buffer system.

In another alternative embodiment, the concentration of the pH buffer is 2-50 mM, e.g., 5-30 mM or 8-20 mM.

In another alternative embodiment, the concentration of the soluble divalent metal salt in the soluble salt substance is 0.2-8 mM, e.g., 0.5-5 mM or 0.8-2 mM.

In another alternative embodiment, the concentration (w/v) of the sucrose is 0.5%-20%, e.g., 0.8%-10% or 0.9%-5%, based on the total weight/volume percentage of the buffer system.

In another alternative embodiment, the concentration (w/v) of the mannitol is 0.5%-20%, e.g., 1%-10% or 2%-5%, based on the total weight/volume percentage of the buffer system.

In another alternative embodiment, the concentration (w/v) of the glycerol is 0.5%-20%, e.g., 1%-10% or 2%-5%, based on the total weight/volume percentage of the buffer system.

In another alternative embodiment, the buffer system has an osmolarity of 280-330 mosm/kg.

In another alternative embodiment, the solvent of the buffer system is an organic solvent or an inorganic solvent; optionally, the solvent is an inorganic solvent, for example, the solvent of the buffer system is water.

In another alternative embodiment, the buffer system is further useful for one or more uses selected from the following:

(a) preventing a virus from undergoing capsid protein fragmentation, aggregation, and damage to packaging integrity caused by low temperature, refrigeration, room temperature, and repeated freeze-thaw cycles;
(b) dispersing a greater number of virus particles;
(c) ensuring isotonicity, enabling more application scenarios, and avoiding adverse reactions caused by high osmolarity.

In a second aspect, the present disclosure provides use of the buffer system according to the first aspect of the present disclosure in the manufacture of a reagent or kit for improving the stability and dispersibility of virus particles.

In another alternative embodiment, the reagent or kit is further useful for one or more uses selected from the following:

(a) preventing a virus from undergoing capsid protein fragmentation, aggregation, and damage to packaging integrity caused by low temperature, refrigeration, room temperature, and repeated freeze-thaw cycles;
(b) dispersing a greater number of virus particles;
(c) ensuring isotonicity, enabling more application scenarios, and avoiding adverse reactions caused by high osmolarity.

In a third aspect, the present disclosure provides a kit, wherein the kit comprises:

i) one or more containers containing the same or different soluble salt substances;
ii) one or more containers containing the same or different polyols;
iii) one or more containers containing the same or different pH buffers;
iv) optionally, one or more containers containing the same or different surfactants.

In another alternative embodiment, the soluble salt substance comprises a soluble monovalent metal salt.

In another alternative embodiment, the soluble monovalent metal salt comprises a soluble sodium salt.

In another alternative embodiment, the soluble salt substance comprises a soluble divalent metal salt.

In another alternative embodiment, the soluble divalent metal salt comprises one or more of a soluble calcium salt, a soluble magnesium salt, a soluble zinc salt, a soluble copper salt, a soluble manganese salt, and a soluble chromium salt.

In another alternative embodiment, the polyol is selected from sucrose, mannitol, and glycerol.

In another alternative embodiment, the surfactant is selected from the following: F68, Tween20, or a combination thereof.

In another alternative embodiment, the kit is further useful for one or more uses selected from the following:

(a) preventing a virus from undergoing capsid protein fragmentation, aggregation, and damage to packaging integrity caused by low temperature, refrigeration, room temperature, and repeated freeze-thaw cycles;
(b) dispersing a greater number of virus particles;
(c) ensuring isotonicity, enabling more application scenarios, and avoiding adverse reactions caused by high osmolarity.

In another alternative embodiment, any two, three, or four (or all) of the above containers may be the same (or one and the same) or different containers.

In another alternative embodiment, any two or three (or all) of the above containers may be the same (or one and the same) or different containers.

In a fourth aspect, the present disclosure provides a method for improving the stability and dispersibility of virus particles, comprising:

providing the buffer system according to the first aspect of the present disclosure and placing the virus particles in the buffer system, thereby improving the stability and dispersibility of the virus particles.

In another alternative embodiment, the virus particles comprise AAV virus particles, lentiviral particles, or a combination thereof.

In a fifth aspect, the present disclosure provides a liquid formulation, wherein the formulation comprises:

(i) a therapeutically effective amount of virus particles;
(ii) a soluble salt substance;
(iii) a polyol;
(iv) a pH buffer,

wherein the pH of the formulation is 7.0-8.0 (e.g., 7.0-7.6 or 7.0-7.4).

In another alternative embodiment, the liquid formulation further comprises a surfactant.

In another alternative embodiment, the soluble salt substance comprises a soluble monovalent metal salt.

In another alternative embodiment, the soluble monovalent metal salt comprises a soluble sodium salt.

In another alternative embodiment, the soluble salt substance comprises a soluble divalent metal salt.

In another alternative embodiment, the soluble divalent metal salt comprises one or more of a soluble calcium salt, a soluble magnesium salt, a soluble zinc salt, a soluble copper salt, a soluble manganese salt, and a soluble chromium salt.

In another alternative embodiment, the polyol is selected from sucrose, mannitol, and glycerol.

In another alternative embodiment, the pH buffer is any one or more selected from the following buffers: PB buffer and HEPES buffer.

In another alternative embodiment, the surfactant is selected from the following: F68, Tween20, or a combination thereof.

In another alternative embodiment, the virus particles comprise AAV virus particles or lentiviral particles.

In another alternative embodiment, the virus particles are AAV virus particles.

In another alternative embodiment, the virus particles are lentiviral particles.

In another alternative embodiment, the virus particles are packaged by an adeno-associated virus vector system.

In another alternative embodiment, the structure of the adeno-associated virus vector system is as described in Patent Application Nos. 202211254858.1 or 202310394646.1.

In another alternative embodiment, the concentration of the virus particles is $5 \times 10^{11}$-$5 \times 10^{14}$ vg/mL, e.g., $2 \times 10^{12}$-$2 \times 10^{14}$ vg/mL or $1 \times 10^{13}$-$1 \times 10^{14}$ vg/mL.

In another alternative embodiment, the formulation has an osmolality of 280-330 mosm/kg.

In another alternative embodiment, the formulation has one or more characteristics selected from the following:

(a) the aggregate content in the formulation is < 1.5%, e.g., < 1.0%, < 0.8%, or < 0.7%;
(b) the free nucleic acid content in the formulation is < 15 ppm, e.g., < 10 ppm or < 8 ppm;
(c) after the formulation is subjected to an accelerated disruption test at 37°C for 7 days, the signal response intensity of virus particles is not significantly decreased, and the viral activity is not significantly reduced;
(d) after the formulation is subjected to an accelerated test for 7 days, the aggregate content is < 1.5%, e.g., < 1.0% or < 0.8%.

In another alternative embodiment, the aggregate content in the formulation is 0.3%-1.5%, e.g., 0.3%-1% or 0.3%-0.7%.

In another alternative embodiment, the free nucleic acid content in the formulation after the accelerated test for 7 days is 25 ppm-35 ppm.

In another alternative embodiment, the aggregate content in the formulation after the accelerated test for 7 days is 0.3%-0.7%. In another alternative embodiment, the formulation comprises:

(a) a therapeutically effective amount of virus particles at a concentration of $5\times10^{11}$-$5\times10^{14}$ vg/mL;
(b) 50-250 mM soluble salt substance;
(c) 0.5%-20% (w/v) polyol;
(d) 2-50 mM pH buffer;

and the pH of the formulation is 7.0-8.0.

In another alternative embodiment, the formulation comprises 0.0005%-0.002% (w/v) surfactant.

In another alternative embodiment, the formulation comprises:

(a) a therapeutically effective amount of virus particles at a concentration of $2\times10^{12}$-$2\times10^{14}$ vg/mL;
(b) 80-200 mM soluble salt substance;
(c) 0.8%-10% (w/v) polyol;
(d) 5-30 mM pH buffer;

and the pH of the formulation is 7.0-7.6.

In another alternative embodiment, the formulation comprises 0.0005%-0.002% (w/v) surfactant.

In another alternative embodiment, the formulation comprises:

(a) a therapeutically effective amount of virus particles at a concentration of $1\times10^{13}$-$1\times10^{14}$ vg/mL;
(b) 100-190 mM soluble salt substance;
(c) 0.9%-5% (w/v) polyol;
(d) 8-15 mM pH buffer;

and the pH of the formulation is7.0-7.4.

In another alternative embodiment, the formulation comprises 0.0008%-0.002% (w/v) surfactant.

In a sixth aspect, the present disclosure provides use of the formulation according to the fifth aspect of the present disclosure in the manufacture of (i) a medicament for preventing and/or treating hereditary coagulation factor deficiency; and/or (ii) a medicament for preventing and/or treating hyperlipidemia-related diseases.

In another alternative embodiment, the hereditary coagulation factor deficiency is hemophilia A, B, or C.

In another alternative embodiment, the hereditary coagulation factor deficiency is mammalian hereditary coagulation factor deficiency; optionally, the hereditary coagulation factor deficiency is human hereditary coagulation factor deficiency.

In another alternative embodiment, the hyperlipidemia-related diseases comprise cardiovascular diseases.

In another alternative embodiment, the cardiovascular diseases comprise atherosclerotic cardiovascular diseases and familial hypercholesterolemia.

In a seventh aspect, the present disclosure provides a pharmaceutical kit, wherein the pharmaceutical kit comprises a container and the formulation according to the fifth aspect of the present disclosure contained within the container.

In an eighth aspect, the present disclosure provides a method for preparing a stable liquid formulation with improved stability and dispersibility of virus particles, comprising the steps of:

(a) mixing a therapeutically effective amount of virus particles, a soluble salt substance, a polyol, and a pH buffer;
(b) adjusting the pH of the resulting mixture to 7.0-8.0 (e.g., 7.0-7.6 or 7.0-7.4);

thereby obtaining a stable liquid formulation with improved stability and dispersibility of virus particles.

In another alternative embodiment, the stable liquid formulation with improved stability and dispersibility of virus particles has one or more characteristics selected from the following:

(a) the aggregate content in the formulation is < 1.5%, e.g., < 1.0% or < 0.8%;
(b) the free nucleic acid content in the formulation is < 15 ppm, e.g., < 10 ppm or < 8 ppm;
(c) after the formulation is subjected to an accelerated disruption test at 37°C for 7 days, the signal response intensity of virus particles is not significantly decreased, and the viral activity is not significantly reduced;

(d) after the formulation is subjected to an accelerated test for 7 days, the aggregate content is < 1.5%, e.g., < 1.0% or < 0.8%.

In another alternative embodiment, the method further comprises the step of mixing the therapeutically effective amount of virus particles, the soluble salt substance, the polyol, and the pH buffer with a surfactant.

It should be understood that within the scope of the present disclosure, the above technical features of the present disclosure and the various technical features specifically described hereinafter (such as in the examples) may be combined with each other to form new or alternative technical solutions. Due to space limitations, detailed descriptions are not provided here one by one.

## DESCRIPTION OF THE DRAWINGS

Figure 1 shows a schematic diagram of the thermal stability assay results for the various formulations of Example 1.
Figure 2 shows the aggregation of the various formulations of Example 1 after 10 freeze-thaw cycles.
Figure 3 shows free nucleic acid levels in the various formulations of Example 1 after 10 freeze-thaw cycles.
Figure 4 shows the AAV full capsid proportions for the various formulations of Example 1 after 10 freeze-thaw cycles.
Figure 5 shows the virus aggregation of the various formulations of Example 1 in the accelerated test.
Figure 6 shows free nucleic acid levels in the various formulations of Example 1 in the accelerated test.
Figure 7 shows the full capsid proportions for the various formulations of Example 1 in the accelerated test.
Figure 8 shows the thermal stability of AAV in the various formulations of Example 2.
Figure 9 shows the signal response intensity of monomeric virus particles after 10 freeze-thaw cycles in Example 2.
Figure 10 shows the aggregate proportion after 10 freeze-thaw cycles in Example 2.
Figure 11 shows the free nucleic acid levels after 10 freeze-thaw cycles in Example 2.
Figure 12 shows the full capsid proportion of virus particles after 10 freeze-thaw cycles in Example 2.
Figure 13 shows the response intensity of virus particles after a 7-day accelerated test in Example 2.
Figure 14 shows the virus particle aggregation after a 7-day accelerated test in Example 2.
Figure 15 shows the free nucleic acid levels after a 7-day accelerated test in Example 2.
Figure 16 shows the signal response intensity of monomeric virus particles in the freeze-thaw test of Example 3.
Figure 17 shows the effect of freeze-thaw on AAV purity in the various formulations of Example 3.
Figure 18 shows the response intensity of AAV particles in the various formulations in the accelerated test of Example 3.
Figure 19 shows the effect of the accelerated test on AAV purity in the various formulations of Example 3.
Figure 20 shows the effect of freeze-thaw on the free nucleic acid levels in the various formulations of Example 3.
Figure 21 shows the effect of the accelerated test on the free nucleic acid levels in Example 3.

## SPECIFIC IMPLEMENTATIONS

After extensive and in-depth research, the inventors of the present disclosure found that a buffer system comprising a soluble salt substance, a polyol, and a pH buffer can maintain the pH of a solution at 7.0-8.0 (e.g., 7.0-7.6 or 7.0-7.4), including, but not limited to, 7.0, 7.1, 7.2, 7.3, 7.4, 7.5, 7.6, 7.7, 7.8, 7.9, or 8.0, and can significantly improve the stability and/or dispersibility of virus particles. In addition, a liquid formulation comprising a soluble salt substance, a polyol, and a pH buffer can also significantly improve the stability and/or dispersibility of virus particles and reduce the content of aggregates and the level of free nucleic acids. Meanwhile, the signal response intensity of virus particles is not significantly decreased, the viral activity is not significantly reduced, and the dispersion and the stability of the virus particles can be significantly improved (e.g., enhanced). The inventors have completed the present disclosure on this basis.

As used herein, the term "liquid formulation" refers to a preparation in such a form that allows the active component to retain effective biological activity, and which contains no other components that exhibit unacceptable toxicity to the subject to whom the formulation is administered. The subject includes a mammal, and optionally is a human.

As used herein, the term "therapeutically effective amount" or "effective amount" refers to an amount that is pharmacologically effective, within the scope of the present disclosure, for the prevention or treatment of diseases. The antibody is effective for the treatment of the diseases. The term "treatment" refers to therapeutic treatment as well as prophylactic or preventive measures. Subjects in need of treatment include those already suffering from the relevant conditions, as well as those in need of prevention of the relevant diseases.

As used herein, the term "pH buffer" refers to a solution formulated with a buffer pair consisting of "a weak acid and a salt of its conjugate base" or "a weak base and a salt of its conjugate acid", which is capable of slowing down pH changes when a certain amount of other substances is added. The pH buffer of the present disclosure maintains the pH of a solution at 7.0-8.0 (e.g., 7.0-7.6 or 7.0-7.4). An optional buffer herein is a PB buffer.

As used herein, the term "divalent metal ion" refers to a positively charged stable structure formed by the loss of two electrons from an atom. Common divalent metal ions include $Ca^{2+}$, $Mg^{2+}$, $Zn^{2+}$, $Cu^{2+}$, $Mn^{2+}$, and $Cr^{2+}$. The optional divalent metal ions herein are $Ca^{2+}$ and $Mg^{2+}$.

One objective of the present disclosure is to provide an AAV formulation that balances stability, is suitable for various administration routes, enables high-concentration dosing, and ensures low osmolarity.

In an aspect, the present disclosure provides a buffer system, wherein the buffer system comprises a soluble salt substance, a polyol, and a pH buffer.

In an alternative embodiment, the buffer system comprises a first buffer system; the first buffer system comprises a soluble salt substance, a polyol, and a pH buffer, and the first buffer system has an osmolarity of 280-330 mosm/kg.

In another alternative embodiment, in the first buffer system, the soluble salt substance comprises one or more soluble monovalent metal salts and/or one or more soluble divalent metal salts; the polyol comprises one or more of sucrose, mannitol, and glycerol; the pH buffer is selected from PB buffer and/or HEPES buffer.

In another alternative embodiment, in the first buffer system, the soluble monovalent metal salt comprises a sodium salt, e.g., sodium chloride; the soluble divalent metal salt comprises one or more of $Ca^{2+}$, $Mg^{2+}$, $Zn^{2+}$, $Cu^{2+}$, $Mn^{2+}$, and $Cr^{2+}$, e.g., $Ca^{2+}$ and $Mg^{2+}$.

In another alternative embodiment, in the first buffer system, the soluble salt substance comprises a soluble monovalent metal salt.

In another alternative embodiment, in the first buffer system, the soluble monovalent metal salt comprises a soluble sodium salt.

In another alternative embodiment, in the first buffer system, the soluble salt substance comprises a soluble divalent metal salt.

In another alternative embodiment, in the first buffer system, the soluble divalent metal salt comprises one or more of a soluble calcium salt, a soluble magnesium salt, a soluble zinc salt, a soluble copper salt, a soluble manganese salt, and a soluble chromium salt.

In another alternative embodiment, in the first buffer system, the soluble divalent metal salt is a soluble calcium salt and/or a soluble magnesium salt.

In another alternative embodiment, in the first buffer system, the soluble monovalent metal salt is a sodium salt, and the soluble divalent metal salts are a soluble calcium salt and a soluble magnesium salt.

In another alternative embodiment, in the first buffer system, the polyol comprises one or more of sucrose, mannitol, or glycerol.

In another alternative embodiment, in the first buffer system, the polyol is sucrose and/or glycerol;

In another alternative embodiment, in the first buffer system, the polyol is sucrose.

In another alternative embodiment, in the first buffer system, the soluble monovalent metal salt is a sodium salt, the soluble divalent metal salts are a soluble calcium salt and a soluble magnesium salt, and the polyol is sucrose.

In another alternative embodiment, in the first buffer system, the pH buffer is any one or more selected from the following buffers: PB buffer and/or HEPES buffer.

In another alternative embodiment, in the first buffer system, the pH buffer is a PB buffer.

In another alternative embodiment, in the first buffer system, the soluble monovalent metal salt is a sodium salt, the soluble divalent metal salts are a soluble calcium salt and a soluble magnesium salt, the polyol is sucrose, and the pH buffer is PB buffer.

In another alternative embodiment, in the first buffer system, the $Na^+$ is derived from NaCl.

In another alternative embodiment, in the first buffer system, the $Ca^{2+}$ is derived from $CaCl_2$, $CH_3COOCa$.

In another alternative embodiment, in the first buffer system, the $Mg^{2+}$ is derived from $MgCl_2$, $Mg_2SO_4$.

In another alternative embodiment, in the first buffer system, the concentration of the soluble monovalent metal salt in the soluble salt substance is 50-250 mM, e.g., 80-200 mM, 100-190 mM, or 100-160 mM.

In another alternative embodiment, in the first buffer system, the concentration of the soluble divalent metal salt is 0.2-8 mM, e.g., 0.5-5 mM or 0.8-2 mM.

In another alternative embodiment, in the first buffer system, the concentration of the soluble monovalent metal salt is 50-250 mM, and the concentration of the soluble divalent metal salt is 0.2-8 mM.

In another alternative embodiment, in the first buffer system, the concentration of the soluble monovalent metal salt is 100-160 mM, and the concentration of the soluble divalent metal salt is 0.8-2 mM.

In another alternative embodiment, in the first buffer system, the concentration of the soluble monovalent metal salt is 80-200 mM, and the concentration of the soluble divalent metal salt is 0.5-5 mM.

In another alternative embodiment, in the first buffer system, the concentration (w/v) of the polyol is 0.5%-20%, e.g., 0.8%-10% or 0.9%-5%, based on the total weight/volume percentage of the buffer system.

In another alternative embodiment, in the first buffer system, the concentration (w/v) of the sucrose is 0.5%-20%, e.g., 0.8%-10% or 0.9%-5%, based on the total weight/volume percentage of the buffer system.

In another alternative embodiment, in the first buffer system, the concentration of the soluble monovalent metal salt is 50-250 mM, the concentration of the soluble divalent metal salt is 0.2-8 mM, and the concentration (w/v) of the sucrose is 0.5%-20%.

In another alternative embodiment, in the first buffer system, the concentration of the soluble monovalent metal salt is 80-200 mM, the concentration of the soluble divalent metal salt is 0.5-5 mM, and the concentration (w/v) of the sucrose is 0.8%-10%.

In another alternative embodiment, in the first buffer system, the concentration of the soluble monovalent metal salt is 100-160 mM, the concentration of the soluble divalent metal salt is 0.8-2 mM, and the concentration (w/v) of the sucrose is 0.9%-5%.

In another alternative embodiment, in the first buffer system, the concentration (w/v) of the mannitol is 0.5%-20%, e.g., 1%-10% or 2%-5%, based on the total weight/volume percentage of the buffer system.

In another alternative embodiment, in the first buffer system, the concentration (w/v) of the glycerol is 0.5%-20%, e.g., 1%-10% or 2%-5%, based on the total weight/volume percentage of the buffer system.

In another alternative embodiment, in the first buffer system, the concentration of the soluble monovalent metal salt is 50-250 mM, the concentration of the soluble divalent metal salt is 0.2-8 mM, and the concentration (w/v) of the glycerol is 0.5%-20%.

In another alternative embodiment, in the first buffer system, the concentration of the soluble monovalent metal salt is 80-200 mM, the concentration of the soluble divalent metal salt is 0.5-5 mM, and the concentration (w/v) of the glycerol is 1%-10%.

In another alternative embodiment, in the first buffer system, the concentration of the soluble monovalent metal salt is 100-160 mM, the concentration of the soluble divalent metal salt is 0.8-2 mM, and the concentration (w/v) of the glycerol is 2%-5%.

In another alternative embodiment, in the first buffer system, the concentration of the pH buffer is 2-50 mM, e.g., 5-30 mM or 8-20 mM.

In another alternative embodiment, in the first buffer system, the pH of the buffer system is maintained at 7.0-8.0 (e.g., 7.0-7.6 or 7.0-7.4).

In another alternative embodiment, in the first buffer system, the buffer system further comprises a surfactant.

In another alternative embodiment, in the first buffer system, the surfactant is selected from one or more of polyoxyethylene-polyoxypropylene ether block copolymer (Poloxamer 188, F68), polysorbate (Tween), polyethylene glycol (PEG), polyethylene glycol p-isooctylphenyl ether (Triton), sorbitan fatty acid ester (Span), sucrose fatty acid ester (SE), polyoxyethylene fatty alcohol ether (Brij), and polyoxyethylene fatty acid ester (Myeij).

In another alternative embodiment, in the first buffer system, the surfactant is selected from the following: F68, Tween, or a combination thereof.

In another alternative embodiment, in the first buffer system, the Tween comprises Tween20.

In another alternative embodiment, in the first buffer system, the concentration (w/v) of the surfactant is 0.0005%-0.002%, e.g., 0.0008%-0.002%, based on the total weight/volume percentage of the buffer system.

In another alternative embodiment, in the first buffer system, the solvent of the buffer system is an organic solvent or an inorganic solvent; optionally, the solvent is an inorganic solvent, for example, the solvent of the buffer system is water.

In another alternative embodiment, the buffer system comprises a second buffer system; the second buffer system comprises a soluble monovalent metal salt, a soluble divalent metal salt with complexing activity, a polyol, and a pH buffer.

In another alternative embodiment, in the second buffer system, the soluble monovalent metal salt comprises a sodium salt, e.g., sodium chloride; the soluble divalent metal salt comprises one or more of $Ca^{2+}$, $Mg^{2+}$, $Zn^{2+}$, $Cu^{2+}$, $Mn^{2+}$, and $Cr^{2+}$, e.g., $Ca^{2+}$ and $Mg^{2+}$; the polyol comprises one or more of sucrose, mannitol, and glycerol; the pH buffer is selected from PB buffer and/or HEPES buffer.

In another alternative embodiment, in the second buffer system, the soluble monovalent metal salt is a sodium salt, and the soluble divalent metal salts are a soluble calcium salt and a soluble magnesium salt.

In another alternative embodiment, in the second buffer system, the soluble monovalent metal salt is a sodium salt, and the soluble divalent metal salts are a soluble calcium salt and a soluble magnesium salt.

In another alternative embodiment, in the second buffer system, the soluble monovalent metal salt is a sodium salt, the soluble divalent metal salts are a soluble calcium salt and a soluble magnesium salt, and the polyol is sucrose.

In another alternative embodiment, in the second buffer system, the soluble monovalent metal salt is a sodium salt, the soluble divalent metal salts are a soluble calcium salt and a soluble magnesium salt, the polyol is sucrose, and the pH buffer is PB buffer.

In another alternative embodiment, in the second buffer system, the concentration of the soluble monovalent metal salt is 50-250 mM, e.g., 80-200 mM, 100-190 mM, or 100-160 mM; the concentration of the soluble divalent metal salt is 0.2-8 mM, e.g., 0.5-5 mM or 0.8-2 mM; the concentration of the polyol is 0.5%-20%, e.g., 0.8%-10% or 0.9%-5%, based on the total weight/volume percentage of the buffer system; the concentration of the pH buffer is 2-50 mM, e.g., 5-30 mM or 8-20 mM; optionally, the pH of the buffer system is 7.0-8.0, e.g., 7.0-7.6 or 7.0-7.4.

In another alternative embodiment, in the second buffer system, the soluble monovalent metal salt is a sodium salt, the soluble divalent metal salts are a soluble calcium salt and a soluble magnesium salt, the concentration of the soluble monovalent metal salt is 50-250 mM, and the concentration of the soluble divalent metal salts is 0.2-8 mM.

In another alternative embodiment, in the second buffer system, the soluble monovalent metal salt is a sodium salt, the soluble divalent metal salts are a soluble calcium salt and a soluble magnesium salt, the concentration of the soluble monovalent metal salt is 100-160 mM, and the concentration of the soluble divalent metal salts is 0.8-2 mM.

In another alternative embodiment, in the second buffer system, the soluble monovalent metal salt is a sodium salt, the soluble divalent metal salts are a soluble calcium salt and a soluble magnesium salt, the concentration of the soluble monovalent metal salt is 80-200 mM, and the concentration of the soluble divalent metal salts is 0.5-5 mM.

In another alternative embodiment, in the second buffer system, the soluble monovalent metal salt is a sodium salt, the soluble divalent metal salts are a soluble calcium salt and a soluble magnesium salt, the polyol is sucrose, the concentration of the soluble monovalent metal salt is 50-250 mM, the concentration of the soluble divalent metal salts is 0.2-8 mM, and the concentration of the polyol is 0.5%-20%.

In another alternative embodiment, in the second buffer system, the soluble monovalent metal salt is a sodium salt, the soluble divalent metal salts are a soluble calcium salt and a soluble magnesium salt, the polyol is sucrose, the concentration of the soluble monovalent metal salt is 100-160 mM, the concentration of the soluble divalent metal salts is 0.8-2 mM, and the concentration of the polyol is 0.8%-10%.

In another alternative embodiment, the soluble monovalent metal salt is a sodium salt, the soluble divalent metal salts are a soluble calcium salt and a soluble magnesium salt, the polyol is sucrose, the concentration of the soluble monovalent metal salt is 80-200 mM, the concentration of the soluble divalent metal salts is 0.5-5 mM, and the concentration of the polyol is 0.9%-5%.

In another alternative embodiment, the second buffer system further comprises a surfactant, wherein the surfactant comprises one or more of polyoxyethylene-polyoxypropylene ether block copolymer (Poloxamer 188, F68), polysorbate (Tween), polyethylene glycol (PEG), polyethylene glycol p-isooctylphenyl ether (Triton), sorbitan fatty acid ester (Span), sucrose fatty acid ester (SE), polyoxyethylene fatty alcohol ether (Brij), and polyoxyethylene fatty acid ester (Myeij), e.g., F68.

In another alternative embodiment, in the second buffer system, the concentration of the surfactant is 0.0005%-0.002% (w/v).

In another aspect, the present disclosure provides use of the aforementioned first buffer system or second buffer system in the manufacture of a reagent or kit for improving the stability and dispersibility of virus particles.

In another alternative embodiment, said improving the stability and dispersibility of virus particles comprises one or more of the following:

(i) preventing a virus from undergoing capsid protein fragmentation, aggregation, or damage to packaging integrity during low-temperature storage, room-temperature storage, or repeated freeze-thaw cycles;
(ii) dispersing a greater number of virus particles;
(iii) having isotonicity, and within a lower isotonic osmolarity range, avoiding adverse reactions caused by high osmolarity, which is beneficial for the product to be used in more application scenarios.

In another alternative embodiment, the virus comprises an AAV virus, a lentivirus, or a recombinant AAV virus; the serotypes of the AAV virus or recombinant AAV virus comprise AAV2, AAV4, AAV5, AAV6, AAV6.2FF, AAV8, AAV9, AAVPHP.eB, AAVPHP.S, or AAVPHP.B.

In another alternative embodiment, the gene of interest carried by the recombinant AAV virus is selected from one or more of the following: tyrosine hydroxylase (TH) gene, GTP cyclohydrolase I (GCH1) gene, aromatic amino acid dopa decarboxylase (AADC) gene, coagulation factor VIII gene, coagulation factor IX gene, or an anti-hyperlipidemic antibody sequence, e.g., Alirocumab or Evolocumab.

In another alternative embodiment, one or more of the soluble salt substances are packaged in the same or different containers, one or more of the polyols are packaged in the same or different containers, and one or more of the pH buffers are packaged in the same or different containers.

In another aspect, the present disclosure provides a kit, wherein the kit comprises:

(i) one or more containers containing the same or different soluble salt substances;
(ii) one or more containers containing the same or different polyols;
(iii) one or more containers containing the same or different pH buffers;
(iv) optionally, one or more containers containing the same or different surfactants.

Optionally, the combination of the soluble salt substance, the polyol, the pH buffer, and the surfactant is selected from the aforementioned first buffer system or second buffer system.

In another alternative embodiment, the kit is further useful for one or more uses selected from the following:

(a) preventing a virus from undergoing capsid protein fragmentation, aggregation, or damage to packaging integrity during low-temperature storage, room-temperature storage, or repeated freeze-thaw cycles;
(b) dispersing a greater number of virus particles;
(c) having isotonicity, and within a lower isotonic osmolarity range, avoiding adverse reactions caused by high osmolarity, which is beneficial for the product to be used in more application scenarios.

In another alternative embodiment, any two, three, or four (or all) of the above containers may be the same (or one and the same) or different containers.

In another alternative embodiment, any two or three (or all) of the above containers may be the same (or one and the same) or different containers.

In another aspect, the present disclosure provides a method for improving the stability and/or dispersibility of virus particles, comprising:
providing the buffer system (the first buffer system or the second buffer system) according to the aforementioned aspect of the present disclosure and placing the virus particles in the buffer system, thereby improving the stability and/or dispersibility of the virus particles.

In another alternative embodiment, the virus particles comprise AAV virus particles, lentiviral particles, or a combination thereof.

In a fifth aspect, the present disclosure provides a liquid formulation, wherein the formulation comprises:

(i) virus particles;
(ii) a soluble salt substance;
(iii) a polyol;
(iv) a pH buffer.

In another alternative embodiment, the liquid formulation comprises virus particles and the buffer system (the first buffer system or the second buffer system) according to the aforementioned aspect.

In another alternative embodiment, the virus particles comprise a therapeutically effective amount of virus particles.

In another alternative embodiment, the virus comprises an AAV virus, a lentivirus, or a recombinant AAV virus; the serotypes of the AAV virus or recombinant AAV virus comprise AAV2, AAV4, AAV5, AAV6, AAV6.2FF, AAV8, AAV9, AAVPHP.eB, AAVPHP.S, or AAVPHP.B.

In another alternative embodiment, the gene of interest carried by the recombinant AAV virus is selected from one or more of the following: tyrosine hydroxylase (TH) gene, GTP cyclohydrolase I (GCH1) gene, aromatic amino acid dopa decarboxylase (AADC) gene, coagulation factor VIII gene, coagulation factor IX gene, or an anti-hyperlipidemic antibody sequence, e.g., Alirocumab or Evolocumab.

In another alternative embodiment, the virus particles are packaged by an adeno-associated virus vector system.

In another alternative embodiment, the structure of the adeno-associated virus vector system is as described in Patent Application No. PCT/CN2022/115831 or CN202310394646.1.

In another alternative embodiment, the concentration of the virus particles is $5\times10^{11}$-$5\times10^{14}$ vg/mL, e.g., $2\times10^{12}$-$2\times10^{14}$ vg/mL or $1\times10^{13}$-$1\times10^{14}$ vg/mL.

In another alternative embodiment, the liquid formulation has an osmolality of 280-330 mosm/kg.

In another alternative embodiment, the liquid formulation has one or more characteristics selected from the following:

(a) the aggregate content in the liquid formulation is < 1.5% (the aggregate content refers to a manifestation of product purity; in SEC chromatographic detection, three signals can be resolved: AAV monomers, AAV aggregates, and free nucleic acids, where the percentage of aggregates refers to the percentage of the aggregate signal relative to all the above signals), e.g., < 1.0%, < 0.8%, or < 0.7%;
(b) the free nucleic acid content in the liquid formulation is < 15 ppm, e.g., < 10 ppm or < 8 ppm;
(c) after the liquid formulation is subjected to an accelerated disruption test at 37°C for 7 days, the signal response intensity of virus particles is not significantly decreased, and the viral activity is not significantly reduced;
(d) after the liquid formulation is subjected to an accelerated disruption test at 37°C for 7 days, the aggregate content is < 1.5%, e.g., < 1.0% or < 0.8%.

In another alternative embodiment, the aggregate content in the liquid formulation is 0.3%-1.5%, e.g., 0.3%-1% or 0.3%-0.7%.

In another alternative embodiment, the free nucleic acid content in the liquid formulation after the accelerated disruption test at 37°C for 7 days is 25 ppm-35 ppm.

In another alternative embodiment, the aggregate content in the liquid formulation after the accelerated disruption test at 37°C for 7 days is 0.3%-0.7%.

In another alternative embodiment, the liquid formulation comprises:

(a) a therapeutically effective amount of virus particles at a concentration of $5 \times 10^{11}$-$5 \times 10^{14}$ vg/mL;
(b) 50-250 mM soluble salt substance;
(c) 0.5%-20% (w/v) polyol;
(d) 2-50 mM pH buffer;

and the pH of the liquid formulation is 7.0-8.0.

In another alternative embodiment, the liquid formulation comprises 0.0005%-0.002% (w/v) surfactant.

In another alternative embodiment, the liquid formulation comprises:

(a) a therapeutically effective amount of virus particles at a concentration of $2 \times 10^{12}$-$2 \times 10^{14}$ vg/mL;
(b) 80-200 mM soluble salt substance;
(c) 0.8%-10% (w/v) polyol;
(d) 5-30 mM pH buffer;

and the pH of the liquid formulation is 7.0-7.6.

In another alternative embodiment, the liquid formulation comprises 0.0005%-0.002% (w/v) surfactant.

In another alternative embodiment, the liquid formulation comprises:

(a) a therapeutically effective amount of virus particles at a concentration of $1 \times 10^{13}$-$1 \times 10^{14}$ vg/mL;
(b) 100-190 mM soluble salt substance;
(c) 0.9%-5% (w/v) polyol;
(d) 8-15 mM pH buffer;

and the pH of the liquid formulation is 7.0-7.4.

In another alternative embodiment, the liquid formulation comprises 0.0008%-0.002% (w/v) surfactant.

In another aspect, the present disclosure provides use of the liquid formulation according to the aforementioned aspect of the present disclosure in the manufacture of (i) a medicament for preventing and/or treating hereditary coagulation factor deficiency; and/or (ii) a medicament for preventing and/or treating hyperlipidemia-related diseases.

In another aspect, the present disclosure provides a method for preventing and/or treating a disease including (i) hereditary coagulation factor deficiency and (ii) hyperlipidemia-related diseases; the method for preventing and/or treating hereditary coagulation factor deficiency comprises administering to a subject a therapeutically effective amount of the liquid formulation according to the aforementioned aspect, wherein the virus particles in the liquid formulation carry a coagulation factor VIII gene and/or a coagulation factor IX gene.

In another alternative embodiment, the mode of administration for the prevention and/or treatment method comprises intravenous injection and/or intramuscular injection.

In another alternative embodiment, the hereditary coagulation factor deficiency is hemophilia A, B, or C.

In another alternative embodiment, the hereditary coagulation factor deficiency is mammalian hereditary coagulation factor deficiency; optionally, the hereditary coagulation factor deficiency is human hereditary coagulation factor deficiency.

In another aspect, the present disclosure provides a pharmaceutical kit, wherein the pharmaceutical kit comprises a container and the liquid formulation according to the aforementioned aspect of the present disclosure contained within the container.

In another aspect, the present disclosure provides a method for preparing a stable liquid formulation with improved stability and/or dispersibility of virus particles, comprising the steps of:

(a) mixing virus particles, a soluble salt substance, a polyol, and a pH buffer;
(b) adjusting the pH of the resulting mixture to 7.0-8.0 (e.g., 7.0-7.6 or 7.0-7.4);

thereby obtaining a stable liquid formulation with improved stability and/or dispersibility of virus particles.

In another alternative embodiment, the steps comprise mixing the components of the liquid formulation according to the aforementioned aspect, and then adjusting the pH of the resulting mixture to 7.0-8.0, e.g., 7.0-7.6 or 7.0-7.4.

In another alternative embodiment, the stable liquid formulation with improved stability and/or dispersibility of virus particles has one or more characteristics selected from the following:

(a) the aggregate content in the liquid formulation is < 1.5%, e.g., < 1.0% or < 0.8%;
(b) the free nucleic acid content in the liquid formulation is < 15 ppm, e.g., < 10 ppm or < 8 ppm;
(c) after the liquid formulation is subjected to an accelerated disruption test at 37°C for 7 days, the signal response intensity of virus particles is not significantly decreased, and the viral activity is not significantly reduced;
(d) after the liquid formulation is subjected to an accelerated disruption test at 37°C for 7 days, the aggregate content is < 1.5%, e.g., < 1.0% or < 0.8%.

In another alternative embodiment, the method further comprises the step of mixing the virus particles, the soluble salt substance, the polyol, and the pH buffer with a surfactant.

In another alternative embodiment, the virus particles are a therapeutically effective amount of virus particles.

**Liquid Formulation**

The liquid formulation with improved stability and dispersibility of virus particles according to the present disclosure mainly comprises:

(i) a therapeutically effective amount of virus particles;
(ii) a soluble salt substance;
(iii) a polyol;
(iv) a pH buffer;

wherein the pH of the liquid formulation is 7.0-8.0 (e.g., 7.0-7.6 or 7.0-7.4).

The therapeutically effective amount of virus particles present in the liquid formulation of the present disclosure is determined by considering the required dosage volume and mode of administration. In the present disclosure, the concentration of the virus particles is $5 \times 10^{11}$-$5 \times 10^{14}$ vg/mL, e.g., $2 \times 10^{12}$-$2 \times 10^{14}$ vg/mL or $1 \times 10^{13}$-$1 \times 10^{14}$ vg/mL, based on the physical titer of the formulation, including, but not limited to, $5 \times 10^{11}$ vg/mL, $6 \times 10^{11}$ vg/mL, $7 \times 10^{11}$ vg/mL, $8 \times 10^{11}$ vg/mL, $9 \times 10^{11}$ vg/mL, $1 \times 10^{12}$ vg/mL, $2 \times 10^{12}$ vg/mL, $3 \times 10^{12}$ vg/mL, $4 \times 10^{12}$ vg/mL, $5 \times 10^{12}$ vg/mL, $6 \times 10^{12}$ vg/mL, $7 \times 10^{12}$ vg/mL, $8 \times 10^{12}$ vg/mL, $9 \times 10^{12}$ vg/mL, $1 \times 10^{13}$ vg/mL, $2 \times 10^{13}$ vg/mL, $3 \times 10^{13}$ vg/mL, $4 \times 10^{13}$ vg/mL, $5 \times 10^{13}$ vg/mL, $6 \times 10^{13}$ vg/mL, $7 \times 10^{13}$ vg/mL, $8 \times 10^{13}$ vg/mL, $9 \times 10^{13}$ vg/mL, $1 \times 10^{14}$ vg/mL, $2 \times 10^{14}$ vg/mL, $3 \times 10^{14}$ vg/mL, $4 \times 10^{14}$ vg/mL, or $5 \times 10^{14}$ vg/mL. The present disclosure includes ranges of values using combinations of any of the above values as upper and/or lower limits.

The buffer system used in the formulation of the present disclosure is a buffer system comprising a soluble salt substance, a polyol, and a pH buffer. In an alternative embodiment, the soluble salt substance of the present disclosure comprises a soluble monovalent metal salt. In an alternative embodiment, the soluble salt substance of the present disclosure comprises a soluble divalent metal salt.

In an alternative embodiment, the soluble monovalent metal salt comprises a soluble sodium salt. In an alternative embodiment, the soluble divalent metal salt comprises one or more of a soluble calcium salt, a soluble magnesium salt, a soluble zinc salt, a soluble copper salt, a soluble manganese salt, and a soluble chromium salt. In the present disclosure, the divalent metal ion in the soluble divalent metal salt may be selected from $Ca^{2+}$ and $mg^{2+}$, and its concentration is 0.2-8 mM, e.g., 0.5-5 mM or 0.8-2 mM, including, but not limited to, 0.2 mM, 0.3 mM, 0.4 mM, 0.5 mM, 0.6 mM, 0.7 mM, 0.8 mM, 0.9 mM, 1.0 mM, 1.5 mM, 2.0 mM, 2.5 mM, 3.0 mM, 3.5 mM, 4.0 mM, 4.5 mM, 5.0 mM, 5.5 mM, 6.0 mM, 6.5 mM, 7.0 mM, 7.5 mM, or 8.0 mM.

In an alternative embodiment, the concentration of the soluble monovalent metal salt in the soluble salt substance in the buffer system is 50-250 mM, e.g., 80-200 mM, 100-190 mM, or 100-160 mM, including, but not limited to, 50 mM, 60 mM, 70 mM, 80 mM, 90 mM, 100 mM, 120 mM, 140 mM, 160 mM, 180 mM, 200 mM, 220 mM, 240 mM, or 250 mM.

In an alternative embodiment, the concentration (w/v) of the polyol in the buffer system is 0.5%-20%, e.g., 0.8%-10% or 0.9%-5%, based on the total weight/volume percentage of the buffer system, including, but not limited to, 0.5%, 0.6%, 0.7%, 0.8%, 0.9%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, or 10.0%.

In an alternative embodiment, the concentration (w/v) of the surfactant in the buffer system is 0.0005%-0.002%, e.g., 0.0008%-0.002%, based on the total weight/volume percentage of the buffer system, including, but not limited to, 0.0005%, 0.0006%, 0.0007%, 0.0008%, 0.0009%, 0.001%, 0.0011%, 0.0012%, 0.0013%, 0.0014%, 0.0015%, 0.0016%, 0.0017%, 0.0018%, 0.0019%, or 0.002%.

In an alternative embodiment, the concentration of the pH buffer in the buffer system is 2-50 mM, e.g., 5-30 mM or 8-20 mM, including, but not limited to, 2 mM, 3 mM, 4 mM, 5 mM, 6 mM, 7 mM, 8 mM, 9 mM, 10 mM, 15 mM, 20 mM, 25 mM, 30 mM, 35 mM, 40 mM, 45 mM, or 50 mM.

The formulation of the present disclosure may optionally be isotonic. The present disclosure includes ranges of values using combinations of any of the above values as upper and/or lower limits.

The amount of surfactant added in the present disclosure is such that it improves viral dispersion while also having an effect of improving viral stability. An optional surfactant in the present disclosure is Poloxamer 188 (F68).

In the present disclosure, the pH of the formulation is adjusted via a buffer system to control the pH within the range of 7.0-8.0 (e.g., 7.0-7.6, or 7.0-7.4). In certain embodiments, the pH of the formulation is between 7.2 and 7.4. The present disclosure includes ranges of values using combinations of any of the above values as upper and/or lower limits.

In addition, through repeated tests, the inventors conducted extensive screening on the components and contents of various buffer systems, and finally obtained the buffer system of the present disclosure from a large number of buffer systems, and found that in this system, the stability and dispersibility of virus particles are significantly improved, and it can also prevent the virus from undergoing capsid protein fragmentation, aggregation, and damage to packaging integrity caused by low temperature, repeated freeze-thaw cycles, refrigeration, and room-temperature storage; disperse a greater number of virus particles; ensure isotonicity, enabling more application scenarios, and avoiding adverse reactions caused by high osmolarity. The buffer system has an osmolarity of 280-340 mosm/kg, e.g., 280-330 mosm/kg, including, but not limited to, 280 mosm/kg, 290 mosm/kg, 300 mosm/kg, 310 mosm/kg, 320 mosm/kg, 330 mosm/kg, or 340 mosm/kg.

The liquid formulation of the present disclosure may comprise one or more additional pharmaceutically acceptable carriers, excipients, or stabilizers, such as those described in Remington's Pharmaceutical Sciences 16th edition, Osol, A. Ed.

(1980), provided that they do not adversely affect the desired characteristics of the formulation. The acceptable carriers, excipients, or stabilizers are non-toxic to recipients at the dosages and concentrations employed, and include other cosolvents; antioxidants, including ascorbic acid and methionine; biodegradable polymers, such as polyesters; and/or salt-forming counterions.

The formulation of the present disclosure may be prepared by combining the various components at certain concentrations using methods well known in the art.

An optional class of methods mainly comprises the following steps: concentrating and buffer-exchanging virus particles into a different formulation buffer by centrifugation (4500 rpm, 4-10°C) using an Ultracel-30K ultrafiltration centrifugal tube, and adjusting the virus particle concentration to a desired concentration with a pH buffer; sterilizing the formulation by filtration using a 0.22 μm Millex syringe filter; packaging the prepared formulation for convenient use, with a vial selected as the packaging material.

The above-mentioned features of the present disclosure, or the features mentioned in the embodiments, may be combined arbitrarily. All features disclosed in this specification may be used in combination with any composition form, and each feature disclosed in this specification may be replaced by any alternative feature that provides the same, equivalent, or similar purpose. Therefore, unless otherwise specified, the disclosed features are only general examples of equivalent or similar features.

**The main advantages of the present disclosure include:**

1. The present disclosure has discovered for the first time that a buffer system comprising a soluble salt substance, a polyol, and a pH buffer can maintain the pH of a solution at 7.0-8.0 (e.g., 7.0-7.6 or 7.0-7.4), and can significantly improve the stability and dispersibility of virus particles. Moreover, a liquid formulation comprising a soluble salt substance, a polyol, and a pH buffer can also significantly improve the stability and dispersibility of virus particles, reduce the contents of aggregates and free nucleic acids, without a significant decrease in the signal response intensity of virus particles and without a significant reduction in the viral activity, and can significantly improve (e.g., enhance) the dispersion and the stability of the virus particles.

2. The buffer system or formulation of the present disclosure can prevent a virus from undergoing capsid protein fragmentation, aggregation, and damage to packaging integrity caused by low temperature, repeated freeze-thaw cycles, refrigeration, and room-temperature storage; disperse a greater number of virus particles; and ensure isotonicity, enabling more application scenarios, and avoiding adverse reactions caused by high osmolarity.

3. The buffer system or formulation of the present disclosure can significantly reduce the content of aggregates, for example, aggregate content < 1.5%, e.g., < 1.0%, < 0.8%, or < 0.7%.

4. The buffer system or formulation of the present disclosure can significantly reduce the content of free nucleic acids, for example, free nucleic acid content < 15 ppm, e.g., < 10 ppm or < 8 ppm.

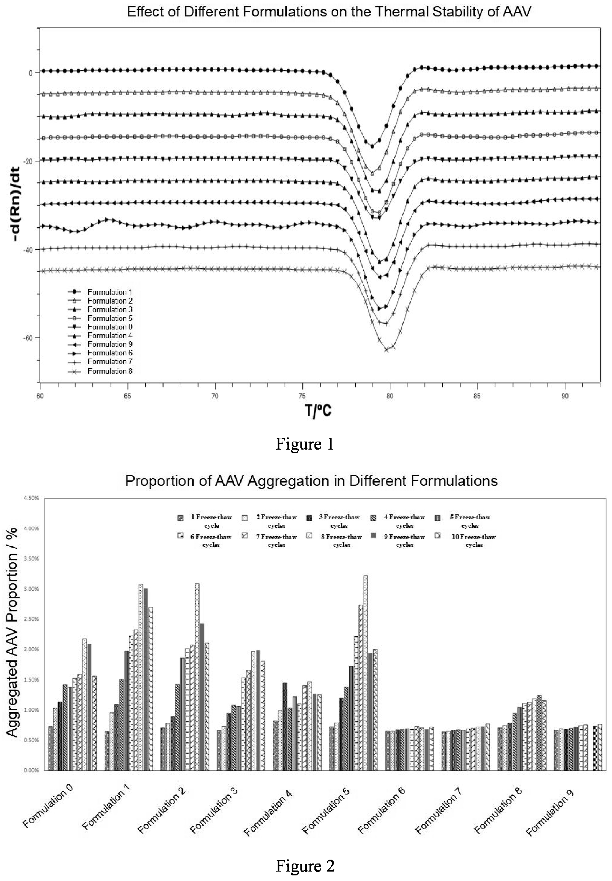

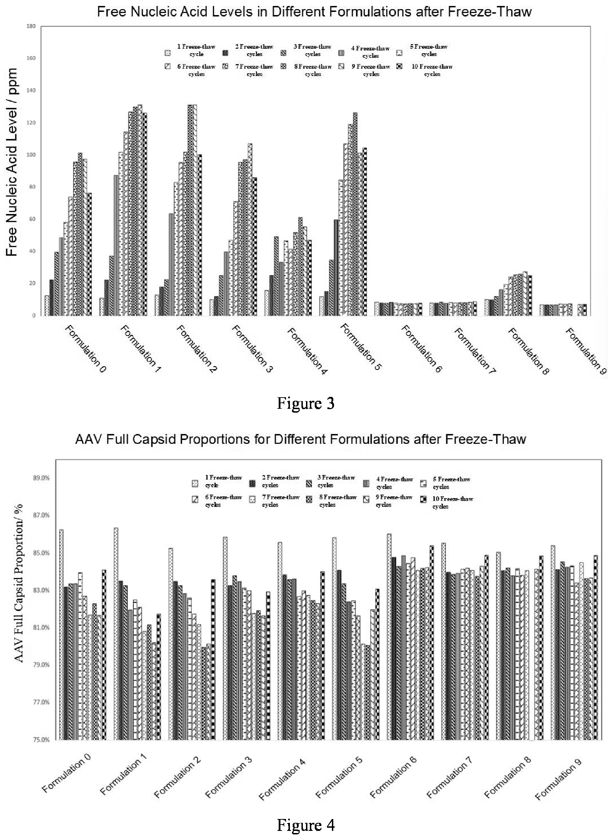

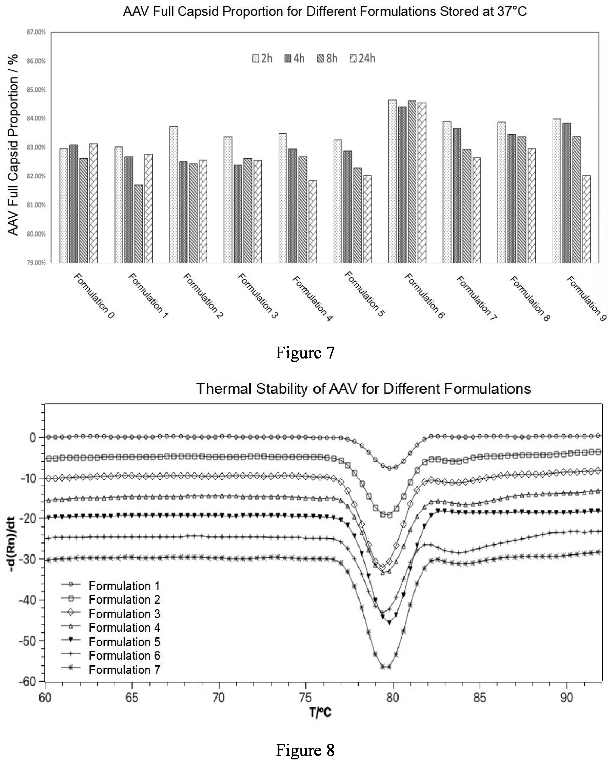

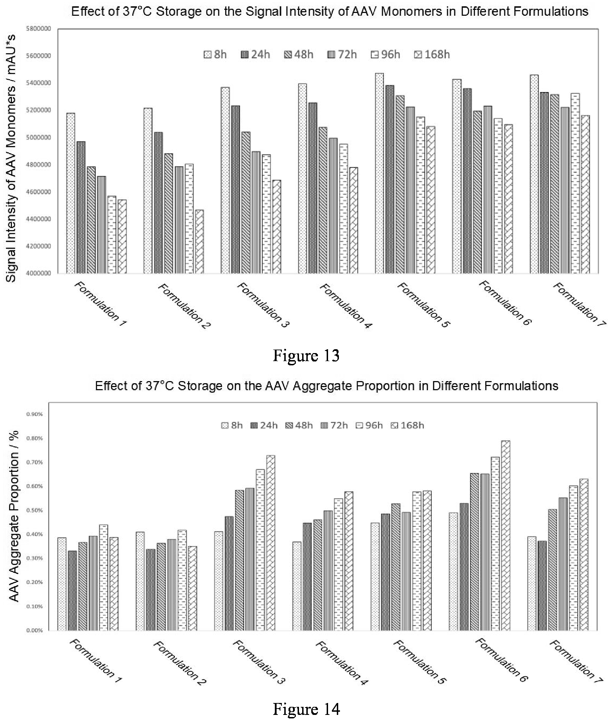

5. The AAV formulation of the present disclosure can effectively prevent a virus from undergoing capsid protein fragmentation and damage to packaging integrity caused by low temperature and repeated freeze-thaw cycles. The formulation shows no obvious decrease in integrity after 10 freeze-thaw cycles, and has better thermal stability compared with traditional formulations (see Figure 1 and Figure 8). It shows no significant genome release at 37°C for at least 24 h (see Figure 3 and Figure 7). Moreover, when the virus particle count is $7-8\times10^{13}$ vg/mL, after 168 hours of continuous disruption at 37°C, it shows no significant damage to the rigid structure of the virus (data from SEC analysis, see Figure 13). After 10 consecutive repeated freeze-thaw cycles and disruption at 37°C, it shows no significant decrease in viral activity (data from activity analysis, see Table 3).

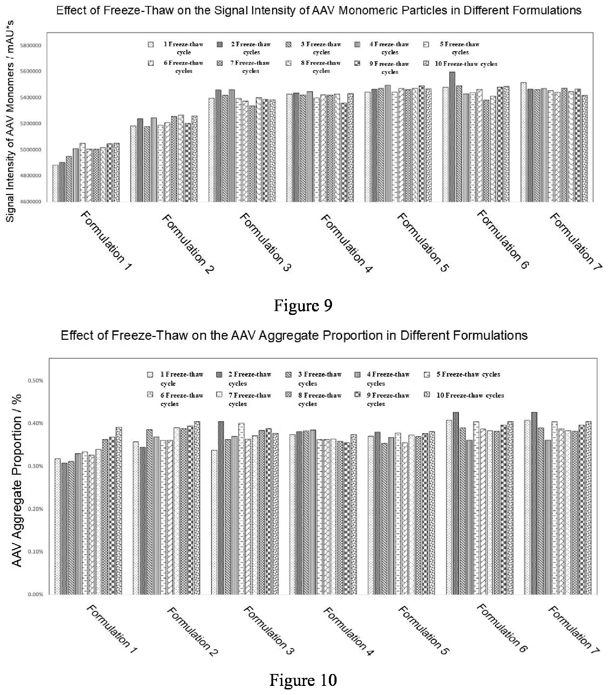

6. The formulation of the present disclosure can disperse a greater number of virus particles. Here, no significant turbidity and precipitation are observed at a loading capacity of $7-8\times10^{13}$ vg/mL (see Figure 9), enabling high-concentration injection.

7. Compared with the existing formulations, on the premise of ensuring stability and loading capacity (physical titer), the formulation of the present disclosure has lower osmolarity and ensures isotonicity (see Table 2), enabling more application scenarios, and avoiding adverse reactions caused by high osmolarity (e.g., high osmolarity associated with Tris buffer can cause pain to patients during injection of the formulation).

The present disclosure is further illustrated below in conjunction with specific examples. It should be understood that these examples are only intended to illustrate the present disclosure and not to limit the scope of the present disclosure. The test methods in the following examples, which are not specified with specific conditions, are generally carried out according to conventional conditions or conditions recommended by the manufacturers. Unless otherwise stated, all percentages and parts are calculated by weight.

Unless otherwise defined, all technical and scientific terms used herein have the same meaning as commonly understood by those skilled in the art. In addition, any methods and materials similar or equivalent to those described can be used in the methods of the present disclosure. The preferred embodiments and materials described herein are exemplary only.

Unless otherwise specified, all materials and reagents used in the examples of the present disclosure are commercially available products.

**General Methods**

**(I) Preparation** of Vectors

AAV useful in the present disclosure is not limited by the use or the gene of interest carried thereby. For example, the AAV can be used for preventing and/or treating hereditary coagulation factor deficiency; and/or (ii) preventing and/or treating hyperlipidemia-related diseases; the hereditary coagulation factor deficiency is hemophilia A, B, or C; the hereditary coagulation factor deficiency is mammalian hereditary coagulation factor deficiency; optionally, the hereditary coagulation factor deficiency is human hereditary coagulation factor deficiency.

The production method of the AAV vector-based formulation is as follows: dispersing the AAV vector obtained after production and purification into a buffer listed in Table 1 or Table 2, performing physical titer detection (PCR method), diluting the stock solution to $8\times10^{13}$ vg/mL (viral concentration) with the corresponding buffer, and subjecting the resulting AAV vector-based formulation to melting curve analysis (differential scanning calorimetry). The results are shown in Figure 1 and Figure 8.

Table 1

| Formulation | PB | NaCl | F68 (w/v) | CaCl$_2$ | Sucrose (w/v) | Mannitol (w/v) | Glycerol (w/v) |
|---|---|---|---|---|---|---|---|
| 0 | 10 mM | 140 mM | N/A | N/A | N/A | N/A | N/A |
| 1 | 10 mM | 140 mM | 0.008% | N/A | N/A | N/A | N/A |
| 2 | 10 mM | 150 mM | 0.005% | N/A | N/A | N/A | N/A |
| 3 | 10 mM | 200 mM | 0.003% | N/A | N/A | N/A | N/A |
| 4 | 10 mM | 250 mM | 0.001% | N/A | N/A | N/A | N/A |
| 5 | 10 mM | 180 mM | 0.001% | N/A | N/A | N/A | N/A |
| 6 | 10 mM | 180 mM | 0.001% | 1 mM | 5% | N/A | N/A |
| 7 | 10 mM | 180 mM | 0.001% | N/A | 5% | N/A | N/A |
| 8 | 10 mM | 180 mM | 0.001% | N/A | N/A | 5% | N/A |
| 9 | 10 mM | 180 mM | 0.001% | N/A | N/A | N/A | 5% |

Table 2

| Formulation | PB | NaCl | F68 (w/v) | CaCl$_2$ | MgCl$_2$ | Sucrose (w/v) |
|---|---|---|---|---|---|---|
| 1 | 10 mM | 110 mM | 0.001% | 1 mM | N/A | 2% |
| 2 | 10 mM | 110 mM | 0.001% | 1 mM | N/A | 3% |
| 3 | 10 mM | 140 mM | 0.001% | 1 mM | N/A | 1% |
| 4 | 10 mM | 140 mM | 0.001% | 1 mM | N/A | 4% |
| 5 | 10 mM | 150 mM | 0.001% | 1 mM | N/A | 3% |
| 6 | 10 mM | 140 mM | 0.001% | N/A | 1 mM | 2% |
| 7 | 10 mM | 140 mM | 0.001% | 1 mM | 1 mM | 2% |

**(II) Analytical Method for Freeze-Thaw Test**

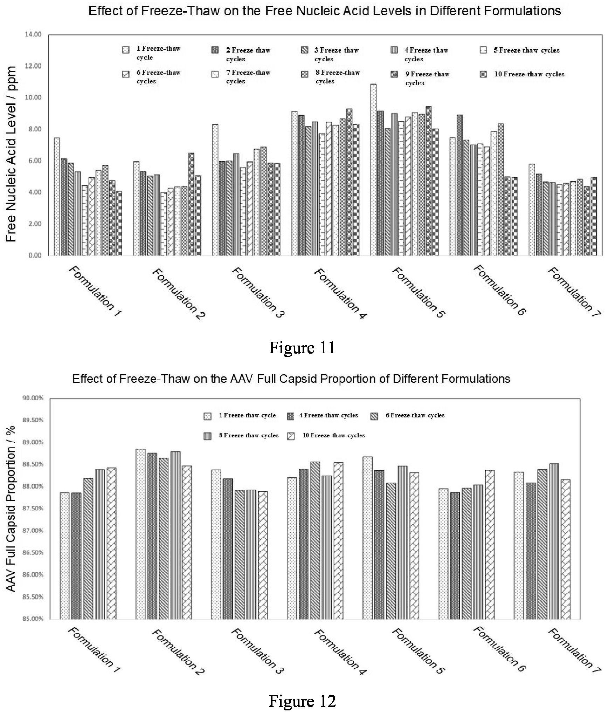

Each formulation group is subjected to 10 freeze-thaw cycles (freezing being performed at -80°C for no less than 6 hours, thawing being performed at 23°C for no less than 30 minutes). After each freeze-thaw cycle, an aliquot of the sample is taken for free nucleic acid assay (by spectrophotometry, results shown in Figure 3 and Figure 11), monomer and aggregate detection (by size-exclusion chromatography, results shown in Figure 2, Figure 9, and Figure 10, where Figure 9 illustrates that the dissolution degree of AAV varies among the various formulations, with higher signal intensity indicating a greater number of virus particles in solution), and AAV integrity detection (by ion-exchange chromatograph, results shown in Figure 4 and Figure 12).

**(III) Analytical Method for Thermal Stability Test**

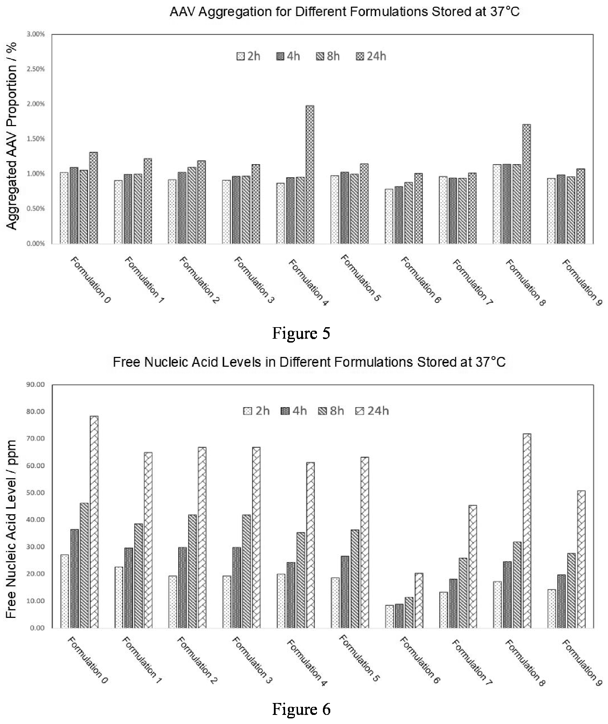

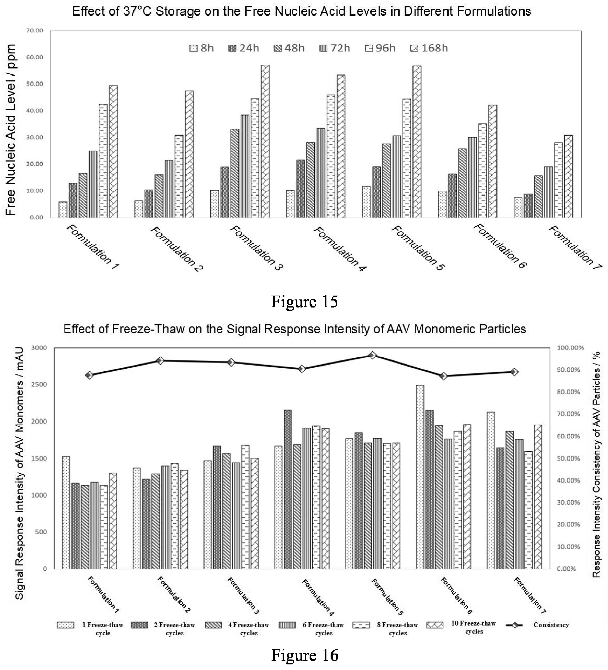

Each formulation group is placed at 37°C for a continuous 168-hour duration. Aliquots of the sample are withdrawn at 8 hours, 24 hours, 48 hours, 72 hours, 96 hours, and 168 hours, respectively, and subjected to free nucleic acid assay (by spectrophotometry, results shown in Figure 6 and Figure 15) and monomer and aggregate detection (by size-exclusion chromatography, results shown in Figure 5, Figure 13, and Figure 14, where Figure 13 indicates that the higher the signal response intensity over time and the more stable its variation, the better the dispersibility and stability of AAV).

The specific standard operating procedures (SOPs) for thermal stability determination, freeze-thaw test, and 37°C accelerated test are all conventional operations.

**(IV) Procedure for Freeze-Thaw Test**

For each formulation, 10 sealed formulation vials (1 mL/vial) are collected and collectively stored at -80°C for a freezing period of not less than 8 hours. Nine vials are then removed and allowed to thaw completely at room temperature for a thawing period of not less than 40 minutes. After complete thawing, the nine vials are collectively stored at -80°C again. Subsequently, the above freeze-thaw cycle is repeated for a total of 9 cycles prior to sampling and detection. All vials are removed, and thawed completely at room temperature, thereby completing 10 freeze-thaw cycles in total.

**(V) Procedure for 37°C Accelerated Disruption Test**

For each formulation, 4-6 sealed formulation vials (1 mL/vial) are collected and collectively placed at 37°C, with timing initiated. One vial of the corresponding formulation is removed at each predetermined time point and then stored at -80°C until sampling and detection.

**(VI) Procedure for SEC Analysis (Aggregate Detection)**

Sample Treatment: No treatment is required; samples are analyzed directly after sampling;
Column: Sepax, STR-500, 4.6×300 mm, 5 $\mu$m, 500 Å;
Eluent: PBS, ethanol;
Detection Wavelengths: 260 nm, 280 nm;
Flow Rate: 0.3 mL/min.

**(VII) Procedure for AEX Analysis (Full Capsid Proportion Assay)**

Sample Treatment: No treatment is required; samples are analyzed directly after sampling;
Column: NanoChrom, 4.6×100 mm, 5 μm, non-porous;
Detection Wavelengths: Excitation wavelength 280 nm, emission wavelength 350 nm;
Flow Rate: 0.8 mL/min;
Eluent: Bis-Tris propane, pH = 9.0, tetramethylammonium chloride.

**(VIII) Free Nucleic Acid Assay**

Sample Treatment: 100 μL of sample/standard control is taken and mixed with 100 μL of 1× ssDNA buffer. The mixture is incubated at room temperature in the dark with shaking for several minutes prior to detection.
Detection Conditions: Excitation wavelength 480 nm, emission wavelength 520 nm.

**(IX) Thermal Stability Assay**

Sample Treatment: 15 μL of test sample with a titer of $1\times10^{11}$ vg/mL is taken and mixed with 5 μL of 1% SYPRO Orange dye, followed by the addition of 25 μL of D-PBS.
Q-PCR Parameter Settings: Temperature is increased from 35°C to 95°C at a ramp rate of 0.05°C/s.
Detection Wavelengths: Excitation wavelength 485 nm, emission wavelength 625 nm.

**(X) Activity Assay**

Sample Treatment: Cells are inoculated with virus at $1\times10^{10}$ vg/well. After 24 hours of inoculation, the cell culture supernatant is collected, added to a microplate pre-coated with an enrichment antibody, and incubated with a labeled detection antibody. All liquid is then discarded, followed by repeated washing, and a chromogenic reagent is added.

**Examples**

The instruments, reagents, and consumables used in the tests were as follows:

**Instruments**

| Instrument Name | Manufacturer | Model |
|---|---|---|
| High-performance liquid chromatography | Agilent | Agilent 1100 |
| High-performance liquid chromatography | Waters | Arc HPLC |
| pH meter | Mettler Toledo | FE20 |
| Electronic balance | Mettler Toledo | CP2102 |
| Micropipette | RAININ | 20/200/1000 μL |

**Reagents and Consumables**

| Reagent | Manufacturer | Cat. No. |
|---|---|---|
| Disodium hydrogen phosphate heptahydrate | Avantor | 7782-85-6 |
| Sodium dihydrogen phosphate monohydrate | Avantor | 10049-21-5 |
| Sodium chloride | Avantor | 7647-14-5 |

(continued)

| Reagent | Manufacturer | Cat. No. |
|---|---|---|
| Sucrose | Sigma | 57-50-1 |
| Glycerol | Xiangerkang | N/A |
| Mannitol | Diamond | 69-65-8 |
| Magnesium chloride hexahydrate | Avantor | 7791-18-6 |
| Calcium chloride dihydrate | Sigma | 101939975 |
| Poloxamer 188 | Gibco | 24040-032 |
| Ultrapure water | Milli-Q | N/A |

**Sample Information**

| Sample Name | Titer |
|---|---|
| Vector 1 (AAV vector for hemophilia) | $8 \times 10^{13}$ vg/mL |

Vector 1 is the product designated KL001 by the Applicant, and its structure is shown in the HA-04 structure diagram in Figure 1 of the patent application (Application No.: CN202310394646.1; Filing Date: 2023-04-13).

**Example 1: Primary Screening of Formulation Combinations**

Vector 1 was buffer-exchanged into buffers of different formulations. After repeated freeze-thaw tests or high-temperature accelerated disruption tests, indicators such as aggregation and integrity were monitored to screen and optimize the optimal formulation conditions.
The types and ratios of the main additives in the candidate formulations were screened, and the drug product formulation was preliminarily determined by detecting aggregation and integrity.

1.1 Test Protocol

| Formulation | PB | NaCl | F68 (w/v) | CaCl$_2$ | Sucrose (w/v) | Mannitol (w/v) | Glycerol (w/v) |
|---|---|---|---|---|---|---|---|
| 0 | 10 mM | 140 mM | N/A | N/A | N/A | N/A | N/A |
| 1 | 10 mM | 140 mM | 0.008% | N/A | N/A | N/A | N/A |
| 2 | 10 mM | 150 mM | 0.005% | N/A | N/A | N/A | N/A |
| 3 | 10 mM | 200 mM | 0.003% | N/A | N/A | N/A | N/A |
| 4 | 10 mM | 250 mM | 0.001% | N/A | N/A | N/A | N/A |
| 5 | 10 mM | 180 mM | 0.001% | N/A | N/A | N/A | N/A |
| 6 | 10 mM | 180 mM | 0.001% | 1 mM | 5% | N/A | N/A |
| 7 | 10 mM | 180 mM | 0.001% | N/A | 5% | N/A | N/A |
| 8 | 10 mM | 180 mM | 0.001% | N/A | N/A | 5% | N/A |
| 9 | 10 mM | 180 mM | 0.001% | N/A | N/A | N/A | 5% |

1.2 Test Results

### 1.2.1 Thermal Stability Results

It can be seen from Figure 1 that the thermal stability of all 10 formulations was approximately 79.5°C. Among them, Formulations 6, 7, 8, and 9 exhibited relatively better performance, with thermal stability all above the average value, which may be attributed to the addition of sugars in the formulations. Meanwhile, the amount of F68 added in Formulations 1, 2, 3, and 4 decreased sequentially, but the thermal stability of the virus increased sequentially, indicating that the amount of F68 added as a surfactant should not be too high, otherwise it would accelerate viral disruption.

### 1.2.2 Freeze-Thaw Test Results

Ten repeated freeze-thaw cycles were performed on each of the 10 formulations. As shown in Figure 2, the aggregation of Formulations 6-9 was significantly less than that of the remaining formulations, indicating that the addition of sugars or polyols in the formulations can significantly reduce the aggregation caused by repeated freeze-thaw cycles. Among them, Formulations 6, 7, and 9 exhibited the least aggregation, while Formulation 8 showed relatively more aggregation. This may be attributed to the fact that mannitol exerts a certain protective effect on the virus under repeated freeze-thaw conditions, but its effect is inferior to that of sucrose and glycerol. A comparison between Formulation 4 and Formulation 5 revealed that increasing the sodium chloride concentration significantly reduced the aggregation, indicating that increasing the sodium chloride concentration helped mitigate viral aggregation, as reflected in Formulations 1 to 4 (where the sodium chloride concentration was gradually increased). A comparison between Formulation 2 and Formulation 5 revealed that their aggregation performance was basically consistent throughout the test. Although the sodium chloride content in Formulation 2 was lower than that in Formulation 5, Formulation 5 did not exhibit less aggregation than Formulation 2. This may be attributed to the effect of F68 in Formulation 2: the F68 content in Formulation 2 was higher than that in Formulation 5, and a relatively high concentration of F68 could better mitigate viral aggregation at a relatively low sodium chloride content.

The free nucleic acid assay results for the various formulations after 10 freeze-thaw cycles are shown in Figure 3. Formulations 6, 7, and 9 exhibited the best performance. Under the protection of sucrose or glycerol, the virus still maintained relatively high integrity after multiple freeze-thaw cycles, with no excessive nucleic acid release. A comparison of Formulations 0 to 5 revealed that increasing the sodium chloride concentration helped stabilize viral integrity, while excessive F68 could lead to viral instability.

The full capsid proportion after 10 freeze-thaw cycles was generally consistent with the performance of the above parameters, and the results are shown in Figure 4, with Formulations 6-9 exhibiting the best performance. A comparison of Formulations 1 to 5 yielded results consistent with the above description: a relatively high concentration of sodium chloride helped stabilize viral integrity against disruption.

### 1.2.3 Accelerated Test Results

In the accelerated test, the various formulations showed no significant differences in aggregation behavior, and the overall performance was relatively consistent. The results are shown in Figure 5.

However, the free nucleic acid performance of the various formulations in the accelerated disruption test was significantly different. As shown in Figure 6, Formulation 6 exhibited the best performance: its free nucleic acid content was markedly lower than that of other formulations during the 24-hour accelerated test, and it exhibited the smallest increase in free nucleic acid content. A comparison between Formulation 6 and Formulation 7 revealed that the addition of calcium ions significantly improved the stability of virus particles.

The full capsid determination results for the various formulations showed that Formulation 6 performed best, as shown in Figure 7, with no significant decline in full capsid proportion over time. In contrast, all other formulations exhibited substantial reductions in both full capsid proportion and full capsid proportion over time.

### 1.2.4 Test Conclusions

After 10 repeated freeze-thaw cycles and a 24 h accelerated test on each of the 10 formulations, Formulation 6 exhibited the best performance, with superior stability in both freeze-thaw and 24 h accelerated tests compared with all other formulations. Therefore, Formulation 6 was selected for further optimization and adjustment. In addition, comparative analysis across multiple formulations revealed that the concentration of F68 added should not be excessively high, because excessive F68 content accelerates the destabilization of virus particles; a moderately high sodium chloride concentration also helps stabilize virus particles, but an excessively high sodium chloride concentration leads to elevated osmolarity and necessitates the addition of sucrose, which further increases formulation osmolarity and is not favorable for large-volume intramuscular injection. Meanwhile, the addition of calcium ions can further enhance the stability of virus particles, which is attributed to the complexation of divalent calcium ions with glutamate residues on the outer surface of virus particles, thereby improving the stability of virus particles. Magnesium ions were also investigated in subsequent tests, and the amounts of sodium chloride, polyols, and divalent ions added should be comprehensively adjusted with consideration of osmolarity requirements.

**Example 2: Secondary Screening of Formulation Combinations**

The addition ratios of key additives in the formulation were screened and confirmed.

2.1 Test Protocol

| Formulation | PB | NaCl | F68 (w/v) | CaCl$_2$ | MgCl$_2$ | Sucrose (w/v) | Osmolality |
|---|---|---|---|---|---|---|---|
| 1 | 10 mM | 110 mM | 0.001% | 1 mM | N/A | 2% | 291 |
| 2 | 10 mM | 110 mM | 0.001% | 1 mM | N/A | 3% | 310 |
| 3 | 10 mM | 140 mM | 0.001% | 1 mM | N/A | 1% | 307 |
| 4 | 10 mM | 140 mM | 0.001% | 1 mM | N/A | 4% | 385 |
| 5 | 10 mM | 150 mM | 0.001% | 1 mM | N/A | 3% | 438 |
| 6 | 10 mM | 140 mM | 0.001% | N/A | 1 mM | 2% | 333 |
| 7 | 10 mM | 140 mM | 0.001% | 1 mM | 1 mM | 2% | 329 |

2.2 Calculation of Magnesium Ion Precipitation Amount

Magnesium ions were added to certain formulations. Given the poor water solubility of magnesium phosphate, the amount of magnesium ions added was calculated as follows:

The formulation pH was controlled at 7.2-7.4, and calculations were performed at pH = 7.3.

$$\mathrm{pKa} = \frac{c\,(H^+)\cdot c\,(PO_4^{3-})}{c\,(HPO_4^{2-})} \quad \dots\dots\dots\dots\dots\dots\dots\dots\dots\dots\dots\dots\dots\dots \quad (1)$$

$$\mathrm{Ksp} = c^2\,(Mg^{2+})\cdot c^3\,(PO_4^{3-}) \quad \dots\dots\dots\dots\dots\dots\dots\dots\dots\dots\dots \quad (2)$$

The second dissociation constant of phosphoric acid has a pKa of 7.2. As derived from Equation (1), $c(PO_4^{3-})$ in this environment is approximately $1\times10^{-7}$ mol/L (the $HPO_4^{2-}$ concentration was calculated at the upper limit of 10 mM). According to Equation (2), when the amount of $Mg^{2+}$ added is 1 mM, the minimum $PO_4^{3-}$ concentration required to generate magnesium phosphate precipitate is $1\times10^{-6}$ mol/L, which is lower than the dissociable $PO_4^{3-}$ concentration under the set conditions. Therefore, no precipitate forms when the amount of $Mg^{2+}$ added is only 1 mM.

2.3 Test Results

Table 3

| Formulation | 1 Freeze-thaw cycle | 10 Freeze-thaw cycles | 37°C 0 h | 37°C 72 h |
|---|---|---|---|---|
| 1 | 83.55 ng/mL | 78.95 ng/mL | 83.55 ng/mL | 84.33 ng/mL |
| 2 | 86.70 ng/mL | 75.78 ng/mL | 86.70 ng/mL | 75.33 ng/mL |
| 3 | 108.38 ng/mL | 110.90 ng/mL | 108.38 ng/mL | 88.08 ng/mL |

(continued)

| Formulation | 1 Freeze-thaw cycle | 10 Freeze-thaw cycles | 37°C 0 h | 37°C 72 h |
|---|---|---|---|---|
| 4 | 110.45 ng/mL | 118.80 ng/mL | 110.45 ng/mL | 96.55 ng/mL |
| 5 | 113.00 ng/mL | 113.78 ng/mL | 113.00 ng/mL | 95.95 ng/mL |
| 6 | 123.10 ng/mL | 122.28 ng/mL | 123.10 ng/mL | 95.40 ng/mL |
| 7 | 112.50 ng/mL | 116.18 ng/mL | 112.50 ng/mL | 91.08 ng/mL |
| Note: Formulations 1-7 in Table 3 correspond to Formulations 1-7 in Table 1 and Table 2, respectively. | | | | |

As shown in Table 3, the freeze-thaw results from the second screening trial indicate that sodium chloride concentration is a relatively important indicator affecting biological activity. Formulations 1 and 2, both having a sodium chloride concentration of 110 mM, exhibited inferior viral activity, suggesting that a moderate increase in sodium chloride concentration helps improve viral activity. In the 72-hour accelerated test at 37°C, Formulation 1 and Formulation exhibited relatively low activity, further supporting that a moderate increase in sodium chloride is beneficial for improving viral activity. The activity test alone did not provide sufficient information, and thus characterization data should be incorporated to identify the optimal formulation.

2.3.1 Thermal Stability Results

The thermal stability of the various formulations was generally consistent, all at 79.8 $\pm$ 0.5°C, with no significant differences among the formulations. The results are shown in Figure 8.

2.3.2 Freeze-Thaw Results

After 10 freeze-thaw cycles, the monomer signal intensity for each formulation showed no obvious trend of variation, but the overall signal intensity differed significantly across formulations. The signal intensities of Formulation 1 and Formulation 2 were significantly lower than those of the other formulations, indicating that the number of virus particles in Formulation 1 and Formulation 2 was considerably lower than that in the other formulations. This is most likely attributable to the fact that 110 mM sodium chloride was insufficient to fully disperse the viral load of $8\times10^{13}$ vg/mL. A comparison between Formulation 1 and Formulation 2 revealed that a moderate increase in sucrose content facilated better dispersion of the virus particles, as shown in Figure 9.

After repeated freeze-thaw cycles, all formulations exhibited favorable anti-aggregation performance, with no extensive aggregation, only minor aggregation observed, and no significant differences among the formulations, as shown in Figure 10.

In the free nucleic acid assay, no severe virus particle disruption was observed for all the test formulations, but relatively significant differences existed among the formulations. Formulation 4 and Formulation 5 performed less favorably, with relatively higher free nucleic acid content. This is most likely due to the higher osmolarity of Formulation 4 and Formulation 5 relative to the other formulations, which compromised the stability of virus particles and resulted in virus particle rupture or nucleic acid leakage. Therefore, it can be seen that a relatively low osmolarity can better maintain the integrity of virus particles, as shown in Figure 11.

Virus particles after 10 freeze-thaw cycles showed no significant differences among the formulations, with generally consistent full capsid proportions, which can be relatively corroborated by the free nucleic acid results, as shown in Figure 12.

2.3.3 Accelerated Test Results

After a 7-day accelerated disruption test at 37°C, the response intensity of virus particles of Formulations 1 to 4 showed a marked declining trend over prolonged acceleration time, while Formulations 5 to 7 performed favorably with no substantial loss observed over prolonged acceleration time. Among them, Formulations 6 and 7 performed optimally, with Formulation 7 slightly better than Formulation 6, indicating that the addition of magnesium ions can better stabilize virus particles against disruption. The results are shown in Figure 13.

After the 7-day accelerated test, all formulations exhibited less aggregation, with aggregation levels not exceeding 1%. Among them, Formulation 1 and Formulation 2 performed the best, maintaining the aggregation levels below 0.5%, as shown in Figure 14.

The various formulations exhibited varying degrees of rupture in the 7-day accelerated disruption test. Free nucleic acid concentrations in all formulations increased over prolonged acceleration time. Among them, Formulation 6 and Formulation 7 performed relatively best, based on a comprehensive evaluation of overall free nucleic acid concentration and the rate of increase in concentration, as shown in Figure 15.

2.3.4 Test Conclusions

Based on evaluations of different ratios of key additives, the amount of sodium chloride to be added depends on the virus particle concentration. Here, for the viral load of $8 \times 10^{13}$ vg/mL in the presenst study, 110 mM sodium chloride was insufficient to fully disperse the virus particles, but this issue could be moderately alleviated by slightly increasing the sucrose concentration. On the other hand, adjusting the contents of sodium chloride and sucrose both exerted a substantial influence on osmolarity. The test results indicated that a relatively high osmolarity was not favorable to the integrity of virus particles. The addition of calcium or magnesium ions effectively stabilized virus particles against disruption, with magnesium ions showing a superior effect. The study further demonstrated that concurrent addition of calcium and magnesium ions (each at 1 mM) further improved the stability. Meanwhile, it is also necessary to investigate whether increasing the magnesium ion content can achieve the desired effect.

**2.3.5 Conclusions**

Based on a series of studies, increasing the addition amount of sodium chloride or sugars can improve the stability and dispersibility of virus particles. Among them, sodium chloride primarily improves the dispersibility of virus particles, while sugars mainly protect virus particles from disruption. The study demonstrates that sucrose provides better protection to virus particles than mannitol and is more convenient for weighing and preparation relative to glycerol. The addition of either sucrose or sodium chloride causes a significant increase in osmolarity, and a relatively high osmolarity is not favorable to the stability of virus particles or to the applicability of the formulation. Therefore, maintaining an isotonic osmolarity is optimal. The amount of the surfactant Poloxamer 188 (F68) needs to be controlled and should not be excessive, since excessive F68 will accelerate viral particle disruption. The study revealed that the addition of either calcium or magnesium ions has a favorable effect on the stability of virus particles, with magnesium ions showing a more pronounced stabilizing effect. However, whether to add magnesium ions and their appropriate amount in the presence of phosphate buffer should be determined on a case-by-case basis. In the current study, the addition of 1 mM magnesium to the 10 mM PB buffer used had no effect, and formulations without magnesium ions also performed well, with no significant aggregation or particle rupture observed.

**Example 3: Optimization of Calcium- and Magnesium-containing Formulations (Supplementary Data Below)**

For AAV vectors of different serotypes carrying different genes of interest, liquid formulations were prepared to improve the stability and dispersibility of the vectors, as specified in Table 3.1.

Table 3.1

| Formulation | Vector Information | PB | NaCl | F68 | CaCl$_2$ | MgCl$_2$ | Sucrose |
|---|---|---|---|---|---|---|---|
| 1 | AAV6.2FF-EGFP | 10 mM | 140 mM | 0.001% | 1 mM | 1 mM | 2% |
| 2 | AAV6.2FF-ScFv | 10 mM | 140 mM | 0.001% | 1 mM | 1 mM | 2% |
| 3 | AAV6.2FF-KL001 | 10 mM | 140 mM | 0.001% | N/A | N/A | 2% |
| 4 | AAV2-EGFP | 10 mM | 140 mM | 0.001% | 1 mM | 1 mM | 2% |
| 5 | AAV8-EGFP | 10 mM | 140 mM | 0.001% | 1 mM | 1 mM | 2% |
| 6 | AAV6.2FF-KL001 | 10 mM | 140 mM | 0.001% | 1 mM | 1 mM | 2% |
| 7 | AAV6.2FF-KL001 | 10 mM | 140 mM | N/A | 1 mM | 1 mM | 2% |

Among them, AAV6.2FF-EGFP refers to the recombinant AAV6.2FF serotype virus carrying the gene of interest EGFP (Enhanced Green Fluorescent Protein), with the nucleotide sequence of the gene of interest EGFP shown in SEQ ID No: 1 and the amino acid sequence thereof shown in SEQ ID No: 2; the vector structure of AAV6.2FF-ScFv corresponds to the construct No. 10: "AAV6.2FF-CAG-UTR-tpa-scFv(EA)-igG4-WPRE" in Figure 5 of the patent application (Application No.: CN202311296135.2; Filing Date: 2023-09-28); the vector structure of KL001 is shown in the HA-04 structure diagram in Figure 1 of the patent application (Application No.: CN202310394646.1; Filing Date: 2023-04-13).

SEQ ID No: 1 (the nucleotide sequence of EGFP)

Atggtgagcaagggcgaggagctgttcaccggggtggtgcccatcctggtcgagctggacggcgacgtaaacg

gccacaagttcagcgtgtccggcgagggcgagggcgatgccacctacggcaagctgaccctgaagttcatctgcaccac

cggcaagctgcccgtgccctggcccaccctcgtgaccaccctgacctacggcgtgcagtgcttcagccgctaccccgac

cacatgaagcagcacgacttcttcaagtccgccatgcccgaaggctacgtccaggagcgcaccatcttcttcaaggacga

cggcaactacaagacccgcgccgaggtgaagttcgagggcgacaccctggtgaaccgcatcgagctgaagggcatcga

cttcaaggaggacggcaacatcctggggcacaagctggagtacaactacaacagccacaacgtctatatcatggccgaca

agcagaagaacggcatcaaggtgaacttcaagatccgccacaacatcgaggacggcagcgtgcagctcgccgaccact

accagcagaacacccccatcggcgacggccccgtgctgctgcccgacaaccactacctgagcacccagtccgccctga

gcaaagaccccaacgagaagcgcgatcacatggtcctgctggagttcgtgaccgccgccgggatcactctcggcatgga

cgagctgtacaagtag.

SEQ ID No: 2 (the amino acid sequence of EGFP)

MVSKGEELFTGVVPILVELDGDVNGHKFSVSGEGEGDATYGKLTLKFICT

TGKLPVPWPTLVTTLTYGVQCFSRYPDHMKQHDFFKSAMPEGYVQERTIFFK

DDGNYKTRAEVKFEGDTLVNRIELKGIDFKEDGNILGHKLEYNYNSHNVYIM

ADKQKNGIKVNFKIRHNIEDGSVQLADHYQQNTPIGDGPVLLPDNHYLSTQS

ALSKDPNEKRDHMVLLEFVTAAGITLGMDELYK.

Multiple performance tests were performed on the above formulations with reference to the procedures described in Examples 1 and 2, and the results are shown in Table 3.2 and Figures. 16-21.

3.1 Effect of Calcium, Magnesium, and F68 on the Thermal Stability of AAV Virus

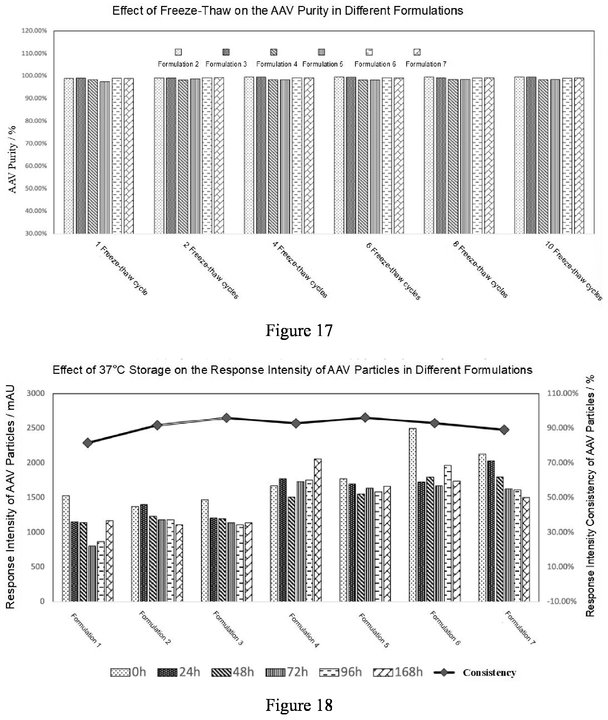

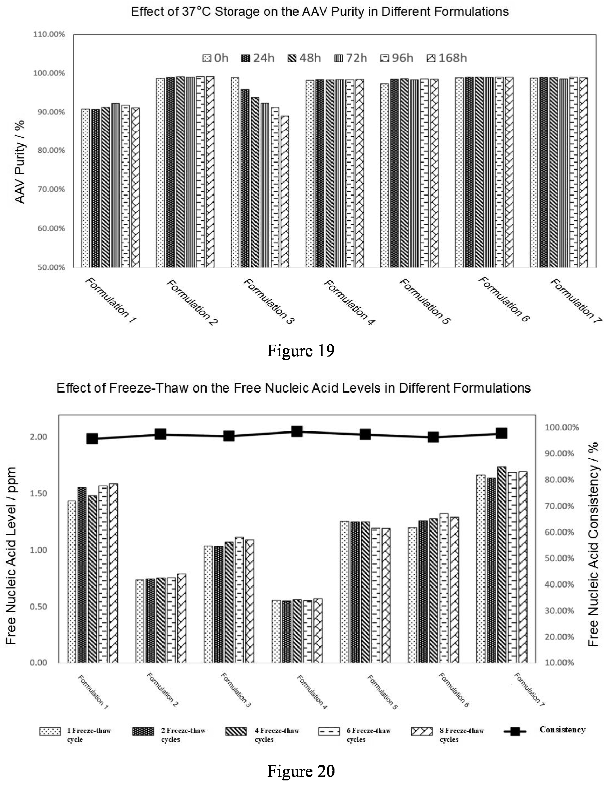

As can be seen from Figure 16, Figure 17, and Figure 20, the addition or omission of calcium ions, magnesium ions, or F68 does not affect the stability of AAV virus particles during repeated freeze-thaw cycles. No significant variations in either AAV virus particle concentration (Figure 16) or AAV virus particle integrity (Figure 17 and Figure 20) were observed among the formulations, and no significant differences were observed in their consistency, indicating that calcium, magnesium, and F68 are not key factors for the stability of virus particles during freeze-thaw cycles.

3.2 Effect of Calcium, Magnesium, and F68 on the Accelerated Test of AAV Virus

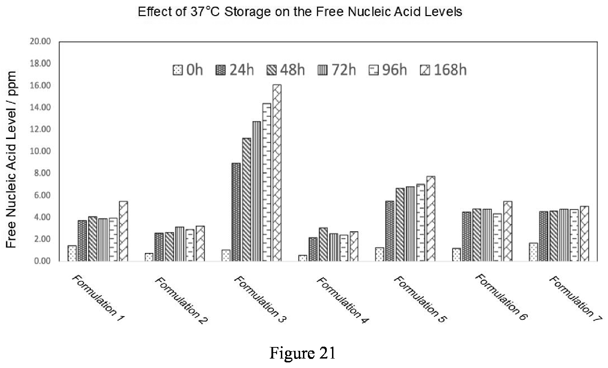

As can be seen from Figure 18, Figure 19, and Figure 21, the addition of calcium or magnesium is critical under accelerated conditions. A comparison of Formulation 2 and Formulation 3 in Figure 19 and Figure 21 reveals that the addition of calcium and magnesium is beneficial to the stability of AAV particles under non-cryogenic conditions. In Figure 19, in formulations without calcium and magnesium, viral purity decreased significantly and continuously during prolonged heat treatment; in Figure 21, during the continuous process, a large amount of free nucleic acid was persistently produced due to viral particle rupture or environmental extrusion, demonstrating that the addition of calcium and magnesium can better stabilize AAV particles and preserve their integrity. A comparison of Formulation 6 and Formulation 7 in Figure 18 reveals that the addition of F68 can improve the dispersibility and adsorption of virus particles to a certain extent, although the effect is not significant.

The results of 10 consecutive freeze-thaw disruption cycles show that the various formulations exhibit good consistency, with no significant differences observed among them, as shown in Table 3.2.

Table 3.2

| Formulation | 1 Freeze-thaw cycle | 2 Freeze-thaw cycles | 4 Freeze-thaw cycles | 6 Freeze-thaw cycles | 8 Freeze-thaw cycles | 10 Freeze-thaw cycles | Consistency |
|---|---|---|---|---|---|---|---|
| 2 | 98.81% | 99.03% | 99.51% | 99.50% | 99.52% | 99.48% | 99.69% |
| 3 | 98.99% | 99.06% | 99.48% | 99.40% | 99.15% | 99.47% | 99.78% |
| 4 | 98.30% | 98.23% | 98.32% | 98.21% | 98.38% | 98.35% | 99.93% |
| 5 | 97.42% | 98.65% | 98.28% | 98.20% | 98.37% | 98.38% | 99.57% |
| 6 | 98.93% | 99.14% | 99.11% | 99.11% | 99.05% | 98.93% | 99.91% |
| 7 | 98.86% | 99.27% | 99.10% | 99.02% | 99.16% | 99.02% | 99.86% |

As can be seen from the above test results, the formulations provided in this example can stabilize the integrity of AAV particles at above 98% during both freeze-thaw processes and 37°C accelerated tests (Formulation 1 had an initial integrity of only about 90%, but its integrity did not decrease during a series of processes, which can also be demonstrated by the RSD data of the results: the RSD values are all greater than 99% under favorable conditions).

3.3 Conclusions

For different AAV6.2FF AAV viruses containing different genes of interest, Formulations 1-2 and 4-7 provided in this example exhibit broad applicability and can stabilize different serotypes and packaged gene vectors. No significant structural damage was observed in the continuous disruption studies. Moreover, the above formulations can stabilize AAV particles for a long term at 2-8°C and for at least several weeks to months at room temperature.
All documents mentioned in the present disclosure are incorporated herein by reference, as if each document were individually incorporated by reference. In addition, it should be understood that after reading the above teachings of the present disclosure, those skilled in the art may make various changes or modifications to the present disclosure, and such equivalent forms shall also fall within the scope defined by the appended claims of the present disclosure.

**Claims**

1. A buffer system, wherein the buffer system comprises a soluble salt substance, a polyol, and a pH buffer.

2. The buffer system according to claim 1, wherein the buffer system comprises a first buffer system; the first buffer system comprises a soluble salt substance, a polyol, and a pH buffer, and the first buffer system has an osmolarity of 280-330 mosm/kg.

3. The buffer system according to claim 2, wherein

   the soluble salt substance comprises one or more soluble monovalent metal salts and/or one or more soluble divalent metal salts;
   the polyol comprises one or more of sucrose, mannitol, and glycerol;
   the pH buffer is selected from PB buffer and/or HEPES buffer.

4. The buffer system according to claim 2 or 3, wherein

   the soluble monovalent metal salt comprises a sodium salt, e.g., sodium chloride;
   the soluble divalent metal salt comprises one or more of $Ca^{2+}$, $Mg^{2+}$, $Zn^{2+}$, $Cu^{2+}$, $Mn^{2+}$, and $Cr^{2+}$, e.g., $Ca^{2+}$ and $Mg^{2+}$.

5. The buffer system according to any one of claims 2 to 4, wherein

   the concentration of the soluble monovalent metal salt is 50-250 mM, e.g., 80-200 mM, 100-190 mM, or 100-160 mM;
   the concentration of the soluble divalent metal salt is 0.2-8 mM, e.g., 0.5-5 mM or 0.8-2 mM;

the concentration of the polyol is 0.5%-20%, e.g., 0.8%-10% or 0.9%-5%, based on the total weight/volume percentage of the buffer system;

the concentration of the pH buffer is 2-50 mM, e.g., 5-30 mM or 8-20 mM;

optionally, the pH of the buffer system is 7.0-8.0, e.g., 7.0-7.6 or 7.0-7.4.

6. The buffer system according to any one of claims 2 to 5, wherein the buffer system further comprises a surfactant, and the surfactant comprises one or more of polyoxyethylene-polyoxypropylene ether block copolymer (Poloxamer 188, F68), polysorbate (Tween), polyethylene glycol (PEG), polyethylene glycol p-isooctylphenyl ether (Triton), sorbitan fatty acid ester (Span), sucrose fatty acid ester (SE), polyoxyethylene fatty alcohol ether (Brij), and polyoxyethylene fatty acid ester (Myeij), e.g., F68.

7. The buffer system according to claim 6, wherein the concentration of the surfactant is 0.0005%-0.002% (w/v).

8. The buffer system according to claim 1, wherein the buffer system comprises a second buffer system; the second buffer system comprises a soluble monovalent metal salt, a soluble divalent metal salt with complexing activity, a polyol, and a pH buffer.

9. The buffer system according to claim 8, wherein

the soluble monovalent metal salt comprises a sodium salt, e.g., sodium chloride;

the soluble divalent metal salt comprises one or more of $Ca^{2+}$, $Mg^{2+}$, $Zn^{2+}$, $Cu^{2+}$, $Mn^{2+}$, and $Cr^{2+}$, e.g., $Ca^{2+}$ and $Mg^{2+}$;

the polyol comprises one or more of sucrose, mannitol, and glycerol;

the pH buffer is selected from PB buffer and/or HEPES buffer.

10. The buffer system according to claim 8 or 9, wherein

the concentration of the soluble monovalent metal salt is 50-250 mM, e.g., 80-200 mM, 100-190 mM, or 100-160 mM;

the concentration of the soluble divalent metal salt is 0.2-8 mM, e.g., 0.5-5 mM or 0.8-2 mM;

the concentration of the polyol is 0.5%-20%, e.g., 0.8%-10% or 0.9%-5%, based on the total weight/volume percentage of the buffer system;

the concentration of the pH buffer is 2-50 mM, e.g., 5-30 mM or 8-20 mM;

optionally, the pH of the buffer system is 7.0-8.0, e.g., 7.0-7.6 or 7.0-7.4.

11. The buffer system according to any one of claims 8 to 10, wherein the buffer system further comprises a surfactant, and the surfactant comprises one or more of polyoxyethylene-polyoxypropylene ether block copolymer (Poloxamer 188, F68), polysorbate (Tween), polyethylene glycol (PEG), polyethylene glycol p-isooctylphenyl ether (Triton), sorbitan fatty acid ester (Span), sucrose fatty acid ester (SE), polyoxyethylene fatty alcohol ether (Brij), and polyoxyethylene fatty acid ester (Myeij), e.g., F68.

12. The buffer system according to claim 11, wherein the concentration of the surfactant is 0.0005%-0.002% (w/v).

13. Use of the buffer system according to any one of claims 1 to 12 in the manufacture of a reagent or kit for improving the stability and dispersibility of virus particles;

optionally, said improving the stability and dispersibility of virus particles comprises one or more of the following:

(i) preventing the virus from undergoing capsid protein fragmentation, aggregation, or damage to packaging integrity during low-temperature storage, room-temperature storage, or repeated freeze-thaw cycles;

(ii) dispersing a greater number of virus particles;

(iii) avoiding adverse reactions caused by high osmolarity;

optionally, the virus comprises an AAV virus, a lentivirus, or a recombinant AAV virus; the serotypes of the AAV virus or recombinant AAV virus comprise AAV2, AAV4, AAV5, AAV6, AAV6.2FF, AAV8, AAV9, AAVPHP.eB, AAVPHP.S, or AAVPHP.B;

optionally, the gene of interest carried by the recombinant AAV virus is selected from one or more of the following: tyrosine hydroxylase (TH) gene, GTP cyclohydrolase I (GCH1) gene, aromatic amino acid dopa decarboxylase (AADC) gene, coagulation factor VIII gene, coagulation factor IX gene, or an anti-hyperlipidemic antibody sequence, e.g., Alirocumab or Evolocumab.

14. The buffer system according to any one of claims 1 to 11, wherein one or more of the components of the buffer system are packaged in the same or different containers.

15. A kit, wherein the kit comprises:

(i) one or more containers containing the same or different soluble salt substances;
(ii) one or more containers containing the same or different polyols;
(iii) one or more containers containing the same or different pH buffers;
(iv) optionally, one or more containers containing the same or different surfactants.

16. A method for improving the stability and dispersibility of virus particles, wherein the method comprises: providing the buffer system according to any one of claims 1 to 11, and placing the virus particles in the buffer system, thereby improving the stability and dispersibility of the virus particles.

17. A liquid formulation, wherein the liquid formulation comprises:

(i) virus particles;
(ii) a soluble salt substance;
(iii) a polyol;
(iv) a pH buffer;
optionally, the liquid formulation comprises virus particles and the buffer system according to any one of claims 1 to 11;
optionally, the virus comprises an AAV virus, a lentivirus, or a recombinant AAV virus; the serotypes of the AAV virus or recombinant AAV virus comprise AAV2, AAV4, AAV5, AAV6, AAV6.2FF, AAV8, AAV9, AAVPHP.eB, AAVPHP.S, or AAVPHP.B;
optionally, the gene of interest carried by the recombinant AAV virus is selected from one or more of the following: tyrosine hydroxylase (TH) gene, GTP cyclohydrolase I (GCH1) gene, aromatic amino acid dopa decarboxylase (AADC) gene, coagulation factor VIII gene, coagulation factor IX gene, or an anti-hyperlipidemic antibody sequence, e.g., Alirocumab or Evolocumab;
optionally, the concentration of the virus particles is $5\times10^{11}$-$5\times10^{14}$ vg/mL, e.g., $2\times10^{12}$-$2\times10^{14}$ vg/mL or $1\times10^{13}$-$1\times10^{14}$ vg/mL;
optionally, the liquid formulation has one or more characteristics selected from the following:

(a) the aggregate content in the liquid formulation is < 1.5%, e.g., < 1.0%, < 0.8%, or < 0.7%;
(b) the free nucleic acid content in the liquid formulation is < 15 ppm, e.g., < 10 ppm or < 8 ppm;
(c) after the liquid formulation is subjected to an accelerated disruption test at 37°C for 7 days, the signal response intensity of virus particles is not significantly decreased, and the viral activity is not significantly reduced;
(d) after the liquid formulation is subjected to an accelerated disruption test at 37°C for 7 days, the aggregate content is < 1.5%, e.g., < 1.0% or < 0.8%.

18. A method for preventing and/or treating a disease, wherein the disease comprises (i) hereditary coagulation factor deficiency and (ii) hyperlipidemia-related diseases;

the method for preventing and/or treating hereditary coagulation factor deficiency comprises administering to a subject a therapeutically effective amount of the liquid formulation according to claim 17, wherein the virus particles in the liquid formulation carry a coagulation factor VIII gene and/or a coagulation factor IX gene;
optionally, the mode of administration for the prevention and/or treatment method comprises intravenous injection and/or intramuscular injection.

19. A pharmaceutical kit, wherein the kit comprises a container and the liquid formulation according to claim 17 contained within the container.

20. A method for preparing a stable liquid formulation with improved stability and dispersibility of virus particles, wherein the method comprises the steps of:

(a) mixing the virus particles, sodium chloride, sucrose, and a pH buffer;
(b) adjusting the pH of the resulting mixture to 7.0-8.0 (preferably 7.0-7.6, more preferably 7.0-7.4);

thereby obtaining a stable liquid formulation with improved stability and dispersibility of virus particles; optionally, mixing the components of the liquid formulation according to claim 17, and then adjusting the pH of the resulting mixture to 7.0-8.0, e.g., 7.0-7.6 or 7.0-7.4.

Effect of Different Formulations on the Thermal Stability of AAV

Figure 1

Proportion of AAV Aggregation in Different Formulations

Figure 2

Figure 3

Figure 4

AAV Aggregation for Different Formulations Stored at 37°C

Figure 5

Free Nucleic Acid Levels in Different Formulations Stored at 37°C

Figure 6

Figure 7

Figure 8

Effect of Freeze-Thaw on the Signal Intensity of AAV Monomeric Particles in Different Formulations

Figure 9

Effect of Freeze-Thaw on the AAV Aggregate Proportion in Different Formulations

Figure 10

Figure 11

Figure 12

Figure 13

Figure 14

Figure 15

Figure 16

Effect of Freeze-Thaw on the AAV Purity in Different Formulations

Figure 17

Effect of 37°C Storage on the Response Intensity of AAV Particles in Different Formulations

Figure 18

Effect of 37°C Storage on the AAV Purity in Different Formulations

Figure 19

Effect of Freeze-Thaw on the Free Nucleic Acid Levels in Different Formulations

Figure 20

Effect of 37°C Storage on the Free Nucleic Acid Levels

Figure 21

## INTERNATIONAL SEARCH REPORT

| International application No. |
| --- |
| **PCT/CN2024/115096** |

| A. | CLASSIFICATION OF SUBJECT MATTER |
| --- | --- |

A61K48/00(2006.01)i;  A61K47/26(2006.01)i;  A61K47/02(2006.01)i;  A61K38/37(2006.01)i;  A61K9/08(2006.01)i;  A61P7/04(2006.01)i;  A61K47/10(2017.01)i

According to International Patent Classification (IPC) or to both national classification and IPC

| B. | FIELDS SEARCHED |
| --- | --- |

Minimum documentation searched (classification system followed by classification symbols)

IPC:  A61K;  A61P

Documentation searched other than minimum documentation to the extent that such documents are included in the fields searched

Electronic data base consulted during the international search (name of data base and, where practicable, search terms used)

CNABS, VEN, CNKI, CNTXT, ENTXTC, ENTXT, DWPI, PUBMED, ISI WEB OF SCIENCE: 4-羟乙基哌嗪乙磺酸, 丙三醇, 病毒, 钙, 甘露醇, 甘油, 缓冲, 磷酸, 氯化钠, 镁, 渗透压, 稳定, 腺病毒, 腺相关病毒, 蔗糖, HEPES, NaCl, sodium chloride, PB, pH, Osmotic pressure, glycerol, Sucrose, Phosphate buffer, mannitol, aav+, 康霖生物科技, 吴昊泉, 姜超, 代勇, 胡晓东, 苏玲玲

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2002041884 A1 (EVANS, R.K. et al.) 11 April 2002 (2002-04-11) claims 1 and 7, and description, paragraphs 12-13 | 1-2, 4-8, 10-20 |
| Y | US 2002041884 A1 (EVANS, R.K. et al.) 11 April 2002 (2002-04-11) claims 1 and 7, and description, paragraphs 12-13 | 3, 9 |
| X | WO 2022133324 A1 (SANGAMO THERAPEUTICS INC. et al.) 23 June 2022 (2022-06-23) description, paragraphs 5-19 and 43 | 1, 8-20 |
| Y | WO 2022133324 A1 (SANGAMO THERAPEUTICS INC. et al.) 23 June 2022 (2022-06-23) description, paragraphs 5-19 and 43 | 3, 9 |
| X | CN 1961961 A (SHENZHEN TSINGHUA YUANXING BIO-PHARM CO., LTD.) 16 May 2007 (2007-05-16) claims 1-10 | 1, 8-20 |

✓ Further documents are listed in the continuation of Box C.    ✓ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 November 2024** | **22 November 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/115096** |

**C.  DOCUMENTS CONSIDERED TO BE RELEVANT**

| Category* | Citation of document, with indication, where appropriate, of the relevant passages | Relevant to claim No. |
|---|---|---|
| X | CN 115337408 A (SHANGHAI XINZHI PHARMACEUTICAL TECHNOLOGY CO., LTD.) 15 November 2022 (2022-11-15) claims 1-13 | 1, 8-20 |
| X | CN 115554418 A (SICHUAN REAL & BEST BIOTECH CO., LTD.) 03 January 2023 (2023-01-03) claims 1-7 | 1, 8-20 |
| X | CN 114728049 A (REGENXBIO INC.) 08 July 2022 (2022-07-08) claims 1-19, and description, paragraph 498 | 1-3, 6-7, 13-20 |

Form PCT/ISA/210 (second sheet) (July 2022)

**INTERNATIONAL SEARCH REPORT**

International application No.

**PCT/CN2024/115096**

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

| International application No. |
| --- |
| **PCT/CN2024/115096** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **18**
because they relate to subject matter not required to be searched by this Authority, namely:

Claim 18 relates to a method for preventing and/or treating a disease, which falls within methods for treatment of diseases as defined in PCT Rule 39.1(iv). Therefore, a search is made on the basis of a pharmaceutical use corresponding to a liquid preparation.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

EP 4 763 230 A1

**INTERNATIONAL SEARCH REPORT**
**Information on patent family members**

International application No.

**PCT/CN2024/115096**

| Patent document cited in search report | | | Publication date (day/month/year) | Patent family member(s) | | | Publication date (day/month/year) |
|---|---|---|---|---|---|---|---|
| US | 2002041884 | A1 | 11 April 2002 | CA | 2799545 | A1 | 13 September 2001 |
| | | | | US | 2004166122 | A1 | 26 August 2004 |
| | | | | US | 7351415 | B2 | 01 April 2008 |
| | | | | EP | 2420247 | A1 | 22 February 2012 |
| | | | | WO | 0166137 | A1 | 13 September 2001 |
| | | | | JP | 2003525909 | A | 02 September 2003 |
| | | | | JP | 5118798 | B2 | 16 January 2013 |
| | | | | EP | 1263461 | A1 | 11 December 2002 |
| | | | | EP | 1263461 | A4 | 12 August 2009 |
| | | | | CA | 2399321 | A1 | 13 September 2001 |
| | | | | CA | 2399321 | C | 30 April 2013 |
| | | | | AU | 4346101 | A | 17 September 2001 |
| WO | 2022133324 | A1 | 23 June 2022 | JP | 2024500133 | A | 04 January 2024 |
| | | | | EP | 4262754 | A1 | 25 October 2023 |
| | | | | CA | 3204462 | A1 | 23 June 2022 |
| | | | | KR | 20230122076 | A | 22 August 2023 |
| | | | | AU | 2021403118 | A1 | 22 June 2023 |
| | | | | AU | 2021403118 | A9 | 18 April 2024 |
| | | | | US | 2024066146 | A1 | 29 February 2024 |
| | | | | IL | 303737 | A | 01 August 2023 |
| | | | | MX | 2023006471 | A | 11 August 2023 |
| CN | 1961961 | A | 16 May 2007 | CN | 1961961 | B | 26 May 2010 |
| CN | 115337408 | A | 15 November 2022 | | None | | |
| CN | 115554418 | A | 03 January 2023 | CN | 115554418 | B | 14 April 2023 |
| CN | 114728049 | A | 08 July 2022 | EP | 4041292 | A1 | 17 August 2022 |
| | | | | TW | 202126319 | A | 16 July 2021 |
| | | | | KR | 20220081365 | A | 15 June 2022 |
| | | | | WO | 2021071835 | A1 | 15 April 2021 |
| | | | | IL | 291930 | A | 01 June 2022 |
| | | | | MX | 2022004146 | A | 19 September 2022 |
| | | | | AR | 120171 | A1 | 02 February 2022 |
| | | | | AU | 2020362119 | A1 | 26 May 2022 |
| | | | | CA | 3156984 | A1 | 15 April 2021 |
| | | | | US | 2024108669 | A1 | 04 April 2024 |
| | | | | JP | 2022552262 | A | 15 December 2022 |
| | | | | BR | 112022006718 | A2 | 12 July 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023111231314
- WO 202211254858 A
- WO 202310394646 A
- CN 2022115831 W
- CN 202310394646
- CN 202311296135

**Non-patent literature cited in the description**

- Remington's Pharmaceutical Sciences